# EUROPÄISCHE PATENTANMELDUNG

(11) **EP 0 579 077 A2**
(43) Veröffentlichungstag der Anmeldung: **19.01.1994**
(21) Anmeldenummer: 93110698.3
(22) Anmeldetag: 05.07.1993
(51) Int. Cl.: C07D 417/14, C07D 417/04, A61K 31/54

(54) **1,3,4-Thiadiazinone zur Behandlung von cardiovaskulären Erkrankungen**

(30) Priorität: 17.07.1992 DE 4223537
(71) Anmelder: MERCK PATENT GmbH, D-64271 Darmstadt (DE)
(72) Erfinder: Jonas, Rochus, Dr., D-64291 Darmstadt (DE); Lues, Ingeborg, Dr., D-64297 Darmstadt (DE); Minck, Klaus-Otto, Dr., D-64372 Ober-Ramstadt (DE); Klockow, Michael, Dr., D-64380 Rossdorf (DE)

(57) **Zusammenfassung**

Thiadiazinone der Formel I 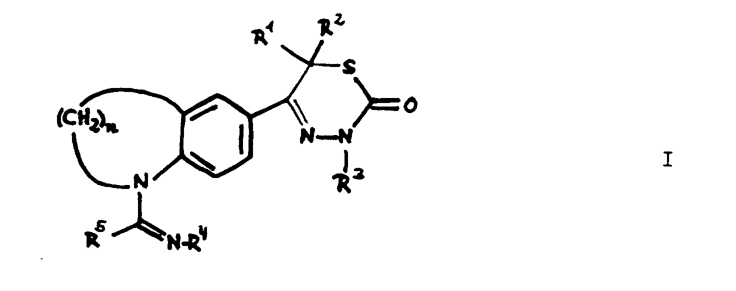
worin
R¹, R², R³, R⁴, R⁵ und n die in Anspruch 1 angegebene Bedeutung haben, zeigen antiarrhythmische Wirkung und eignen sich zur Bekämpfung von kardiovaskulären Erkrankungen.

## Beschreibung

Die Erfindung betrifft neue Thiadiazinonderivate der Formel I 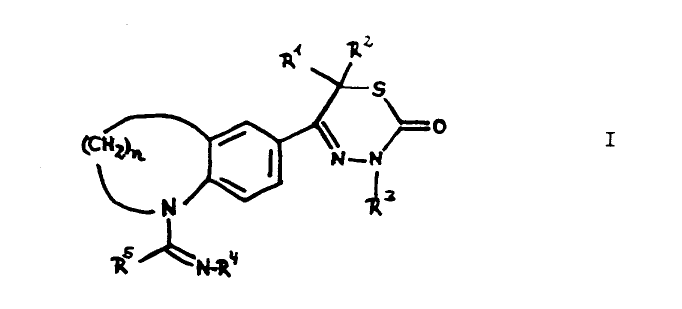
worin
R¹ und R² jeweils unabhängig voneinander H oder A,
_{R}3 H, A oder Ac,
R⁴ H, A, Cycloalkyl mit 3-7 C-Atomen, Ar oder Ar-alk,
_{R}⁵ Ar oder Het,
A Alkyl mit 1-8 C-Atomen,
Ac A-CO-, Ar-CO-, Ar-alk-CO-, A-O-CO- oder A-NH-CO-,
-alk Alkylen mit 1-5 C-Atomen,
Ar einen unsubstituierten oder einen ein-, zwei- oder dreifach durch A, OH, OA, F, Cl, Br, I, SA, SOA, S0₂A, NH₂, NHA, NA₂, NHAc, NHS0₂A, CN oder N0₂ substituierten Phenylrest,
Het einen gesättigten oder ungesättigten 5- oder 6-gliedrigen heterocyclischen Rest mit 1-4 N-, O- und/oder S-Atomen, der ein- oder zweifach durch A, OA, F, Cl,Br, I, OH, N0₂, NH₂, NHA, NA₂, NHAc, NH-S0₂-A, SO-A, S0₂-A, S0₂NH₂ und/oder S0₂NHA, substituiert sein kann
   und
n 2, 3 oder 4

bedeuten,
sowie deren Salze.

Thiadiazinonderivate, deren Grundstruktur der Formel I entspricht, die jedoch ansonsten ein anderes Substitutionsmuster aufweisen, sind aus DE 37 19 031 A1 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I bei guter Verträglichkeit wertvolle pharmakologische Eigenschaften besitzen. Insbesondere zeigen sie eine starke antiarrhythmische Wirkung sowie einen positiv inotropen Effekt; ferner wirken die Substanzen vasodilatierend und fördern daher die Durchblutung. Die vasodilatierende und die Herzwirkung kann z.B. an narkotisierten oder wachen Hunden, Katzen, Affen oder Minischweinen, die positiv inotrope Wirkung auch an isolierten Herzpräparationen (z.B. Vorhof, Papillarmuskel oder perfundiertes Ganzherz) von Ratte, Meerschweinchen, Katze oder Hund ermittelt werden, z.B. nach Methoden, wie sie in Arzneimittelforschung, Band 31 (I) Nr. 1 (1981), Seiten 141 bis 170, oder von Schliep et al. im 9th International Congress of Pharmacol., London, Abstracts of papers 9P, beschrieben sind.

Weiterhin treten antithrombotische, thrombozytenaggregationshemmende und die Erythrozytenform beeinflussende Eigenschaften auf. Die Beeinflussung der Thrombozytenfunktion im Sinne einer Aggregationshemmung kann an der Ratte ex vivo im Test nach Born (Nature 194, 927-929, 1962) nachgewiesen werden. Die antithrombotische Wirkung zeigt sich in der Verlängerung der Blutungszeit nach Stella (Thrombos. Res. 7, 709-716, 1975) in der Verminderung des Thrombusgewichtes bei der kälteinduzierten Thrombosierung der Jugular-Vene bei der Ratte nach Meng (Ther. Ber. 47, 69-79, 1975) und der Erhöhung der zur vollständigen Thrombosierung notwendigen Laserimpulse an der Mesenterial-Venole der Ratte, entsprechend einer Abwandlung der Methode nach Kovacs (Microvasc. Res. 6, 194-201, 1973).

Die günstige Wirkung auf die Erythrozytenverformbarkeit ist im Nucleoporefilter nach Schmid-Schönbein (Pflüger's Archiv 338, 93-114, 1973) nachweisbar. Auch lassen sich günstige Effekte auf die Fibrinolyse/Euglobulinlysiszeit nach v. Kaulla (Progr. Chem. Fibrinol., Thrombol. 1, 131-149, 1975; ed J.F. Davidson, Raven Press, N.Y.) feststellen.

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe dienen.

Gegenstand der Erfindung sind dementsprechend die Verbindungen der Formel I, ihre Säureadditionssalze sowie ein Verfahren zu ihrer Herstellung, dadurch gekennzeichnet, daß man eine Verbindung der Formel II 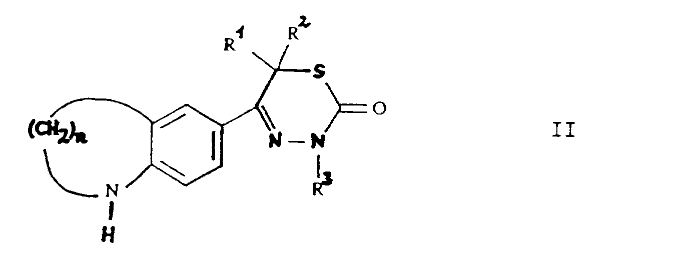
worin
R¹, R², R³ und n die angegebenen Bedeutungen haben, mit einem Imidchlorid der Formel III 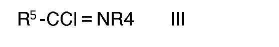
   worin
R⁴ und R⁵ die angegebenen Bedeutungen haben, umsetzt oder daß man ein Keton der Formel IV 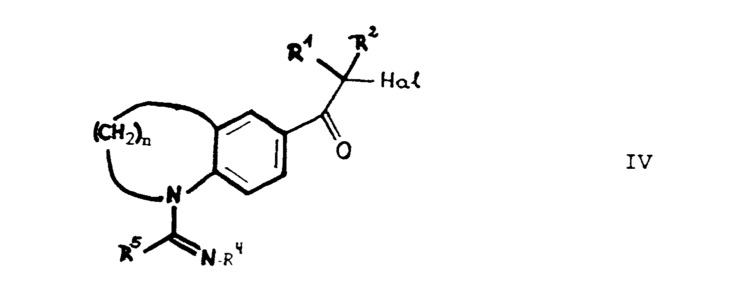
   worin
R¹, R², R⁴, R⁵ und n die angegebenen Bedeutungen haben und Hal Cl, Br oder I bedeutet, mit einer Verbindung der Formel V 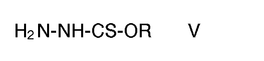
   worin
R Alkyl mit 1-5 C-Atomen oder ein Äquivalent eines Metall- oder Ammoniumkations bedeutet, umsetzt,
oder daß man zur Herstellung einer Verbindung der Formel I, worin R⁴ = H ist, eine entsprechende Verbindung, die jedoch anstelle von R⁴ eine Aminoschutzgruppe trägt, mit einem hydrolysierenden oder hydrogenolysierenden Mittel behandelt
oder daß man zur Herstellung einer Verbindung der Formel I, worin R⁴ = H ist, eine Halogenmagnesiumverbindung der Formel VI 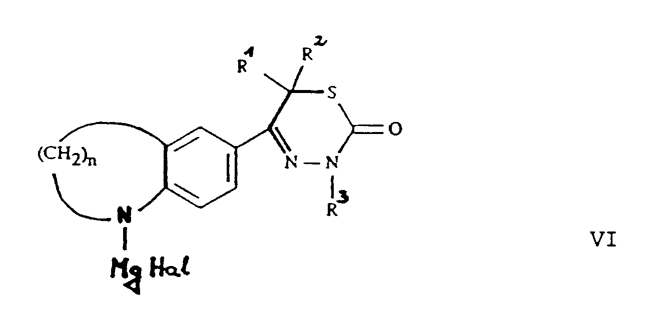
   worin
R¹, R², R³ Hal und n die angegebenen Bedeutungen haben, mit einem Nitril der Formel VII 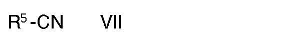
   worin
R⁵ die angegebene Bedeutung hat, umsetzt und das erhaltene Produkt anschließend hydrolysiert, oder daß man eine Verbindung der Formel VIII 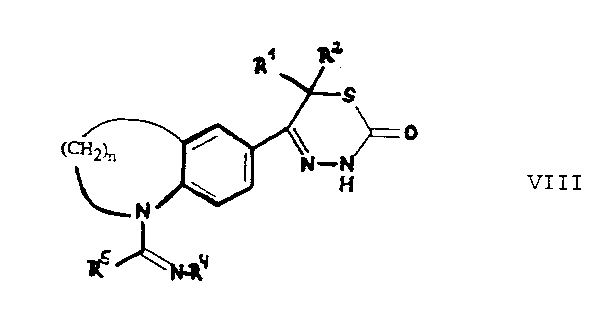
   worin
R¹, R², R⁴, R⁵ und n die angegebenen Bedeutungen haben, mit einem Acyl- oder Alkylderivat der Formel IX 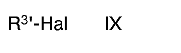
   worin
R3' A oder Ac und
Hal die zuvor angegebene Bedeutung hat, umsetzt,

oder daß man gegebenenfalls in einem Thiadiazinonderivat der Formel I einen oder beide Reste R⁴ und/oder R⁵ in (einen) andere(n) Rest(e) R⁴ und/oder R⁵ umwandelt und/oder eine erhaltene Base der Formel I durch Behandlung mit einer Säure in eines ihrer Säureadditionssalze umwandelt.

Vor- und nachstehend haben R¹, R², R³, R⁴, R⁵, n, Hal und R die bei den Formeln I, IV bzw. V angegebenen Bedeutungen, wenn nicht ausdrücklich etwas anderes angegeben ist.

In den Formeln steht A für Alkylreste mit 1-8 C-Atomen, die bevorzugt unverzweigt sind, vorzugsweise 1, 2, 3, 4 oder 5 C-Atome besitzen, bevorzugt Methyl, ferner bevorzugt Ethyl oder Propyl, weiterhin bevorzugt Isopropyl, Butyl, Isobutyl, sek.-Butyl, tert-Butyl, n-Pentyl oder Isopentyl bedeuten.

Der Rest Ar kann unsubstituiertes Phenyl sein, ist aber bevorzugt einfach oder zweifach substituiertes Phenyl, wobei die Substituenten gleich oder verschieden sein können und vorzugsweise in para-, bzw. in para- und meta-Position stehen. Besonders bevorzugte Substituenten sind Methoxy, Chlor, N0₂, CN und NHS0₂CH₃, aber auch Hydroxy, Alkylthio, Alkylsulfinyl, Alkylsulfonyl und Ethoxy.

Im einzelnen ist Ar bevorzugt Phenyl, p-Nitrophenyl, p-Cyanphenyl, p-Methansulfonamidophenyl, p-Chlorphenyl, p-Methoxyphenyl oder 3,4-Dimethoxyphenyl.

Die Gruppe "-alk" steht für eine geradkettige oder verzweigte Alkylengruppe, vorzugsweise für -CH₂-oder -CH₂-CH₂-.

Cycloalkyl kann 3-7 C-Atome enthalten, besitzt aber bevorzugt 5 oder 6 C-Atome und ist vorzugsweise Cyclopentyl oder Cyclohexyl.

Die Reste R¹, R² und R³ bedeuten vorzugsweise jeweils H oder Methyl.

R³ bedeutet ferner vorzugsweise A-CO-, Ar-CO-, Ar-alk-CO-, A-O-CO- oder A-NH-CO-,worin A, "-alk" sowie Ar die bereits angegebenen bevorzugten Bedeutungen besitzen.

Der Rest R⁴ bedeutet vorzugsweise Ethyl, weiterhin bevorzugt Phenyl, Benzyl oder 2-Phenylethyl, aber auch Cyclopentyl, Methyl, Propyl, Isopropyl, Butyl oder Isopentyl.

Der Rest R⁵ bedeutet bevorzugt Phenyl, besonders bevorzugt substituiertes Phenyl, vorzugsweise p-Nitrophenyl, p-Methansulfonamidophenyl, p-Cyanphenyl, p-Chlorphenyl, p-Methoxyphenyl oder 3,4-Dimethoxyphenyl, ferner bevorzugt 2-Thienyl oder 2-, 3- oder 4-Pyridyl.

Gegenstand der Erfindung sind insbesondere diejenigen Verbindungen der Formel I, in denen mindestens einer der genannten Reste eine der vorstehend angegebenen bevorzugten Bedeutungen hat.

Einige bevorzugte Gruppen von Verbindungen können durch die folgenden Teilfomeln la bis If ausgedrückt werden, die der Formel I entsprechen und worin die nicht näher bezeichneten Reste die bei der Formel I angegebene Bedeutung haben, worin jedoch
in la der Dihydrothiadiazinon-Ring in 6-Stellung eines 1,2,3,4-Tetrahydrochinolins steht,
R¹, R² und R³ jeweils unabänglg voneinander H oder Methyl,
R⁴ Alkyl mit 1-5 C-Atomen und
R⁵ ein- oder zweitfach substituiertes Phenyl bedeuten;
in Ib der Dihydrothiadiazinon-Ring in 6-Stellung eines 1,2,3,4-Tetrahydrochinolins steht,
R¹, R² und R³ jeweils unabhängig voneinander H oder Methyl,
_{R}⁴ Phenyl und
R⁵ ein- oder zweifach substituiertes oder unsubstituiertes Phenyl bedeuten;
in Ic der Dihydrothiadiazinon-Ring in 6-Stellung eines 1,2,3,4-Tetrahydrochinolins steht,
R¹, R² und R³ jeweils unabhängig voneinander H oder Methyl,
_{R}⁴ Ethyl und
R⁵ ein- oder zweifach substituiertes Phenyl bedeuten;
in Id der Dihydrothiadiazinon-Ring in 6-Stellung eines 1,2,3,4-Tetrahydrochinolins steht,
R¹, R² und R³ jeweils unabhängig voneinander H oder Methyl,
R⁴ Alkyl mit 1-5 C-Atomen und
R⁵ Phenyl, p-Nitrophenyl, p-Cyanphenyl, p-Methansulfonamidophenyl, p-Chlorphenyl, p-Methoxyphenyl, 3,4-Dimethoxyphenyl oder 2-Thienyl bedeuten;
in le der Dihydrothiadiazinon-Ring in 6-Stellung eines 1,2,3,4-Tetrahydrochinolins steht,
R¹, R² und R³ jeweils unabhängig voneinander H oder Methyl,
R⁴ Benzyl oder 2-Phenylethyl und
R⁵ Phenyl, p-Nitrophenyl, p-Cyanphenyl, p-Methansulfonamidophenyl, p-Chlorphenyl, P-Methoxyphenyl, 3,4-Dimethoxyphenyl oder 2-Thienyl bedeuten;
in If der Dihydrothiadiazinon-Ring in 6-Stellung eines 1,2,3,4-Tetrahydrochinolins steht,
R¹, R² und R³ jeweils unabhängig voneinander H oder Methyl,
_{R}⁴ Ethyl und
R⁵ 2-Thienyl, p-Nitrophenyl, p-Cyanphenyl, p-Methansulfonamidophenyl

bedeuten.

Die Verbindungen der Formel I werden im übrigen nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, daß man sie aus dem Reaktionsgemisch nicht isoliert, sondern sofort weiter zu den Verbindungen der Formel I umsetzt.

Die Ausgangsstoffe der Formeln II und III sind teilweise bekannt. Sofern sie nicht bekannt sind, können sie nach an sich bekannten Methoden hergestellt werden. Die Herstellung der Verbindungen der Formel II ist aus DE 37 19 031 bekannt.

Im einzelnen erfolgt die Umsetzung der Verbindungen der Formel II mit den Verbindungen der Formel III in Gegenwart oder Abwesenheit eines inerten Lösungsmittels bei Temperaturen zwischen etwa -20 und etwa + 150 _{°} , vorzugsweise zwischen 20 und 1000. Als Lösungsmittel eignen sich beispielsweise Kohlenwasserstoffe wie Benzol, Toluol, Xylole oder Mesitylen; halogenierte Kohlenwasserstoffe wie Dichlormethan, Trichlorethylen oder Chlorbenzol; Alkohole wie Methanol, Ethanol oder Isopropanol; Glykole oder Glykolether wie Ethylenglykol, Diethylenglykol, 2-Methoxyethanol; Nitrile wie Acetonitril; Ether wie Tetrahydrofuran oder Dioxan; Amide wie Dimethylformamid (DMF); Sulfoxide wie Dimethylsulfoxid. Auch Gemische dieser Lösungsmittel sind geeignet.

In den Verbindungen der Formel IV steht Hal bevorzugt für CI oder Br.

In den Verbindungen der Formel V steht R bevorzugt für Methyl oder Ethyl, aber auch für Na, K oder NH₄.

Diese Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt. Andererseits ist es möglich, die Reaktion stufenweise durchzuführen, wobei man weitere Zwischenprodukte isolieren kann.

Die Ausgangsstoffe der Formeln IV und V sind teilweise bekannt. Sofern sie nicht bekannt sind, können sie nach an sich bekannten Methoden hergestellt werden. Die Ketone der Formel IV sind beispielsweise durch Friedel-Crafts-Synthese aus entsprechenden Tetrahydrochinolinderivaten mit Verbindungen der Formel Hal-CO-CHR²⁻Hal zugänglich.

Im einzelnen erfolgt die Umsetzung der Ketone der Formel IV mit den Verbindungen der Formel V unter Bedingungen, wie sie für die Reaktion zwischen Verbindungen der Formeln II und III zuvor angegeben wurden.

Man kann eine Verbindung der Formel 1 auch dadurch erhalten, daß man eine Verbindung, die sonst der Formel I entspricht, aber anstelle von R⁴ eine "Aminoschutzgruppe" trägt, mit einem Reagenz behandelt, welches diese "Schutzgruppe" reduktiv entfernt.

Als "Schutzgruppen" dienen vorzugsweise C0₂-CH₂C₆H₅, besonders bevorzugt OH, welches vorzugsweise durch Übergangsmetallcarbonyle, besonders bevorzugt durch Eisenpentacarbonyl, aber auch durch Fe₂(CO)₉ bei Temperaturen zwischen etwa -20 und etwa + 150 _{°} , vorzugsweise zwischen 20 und 100°, in Gegenwart oder Abwesenheit eines inerten Lösungsmittels, entfernt werden kann.

Als Lösungsmittel eignen sich beispielsweise die oben für die Reaktion von II mit III angegebenen.

Verbindungen der Formel 1 kann man auch dadurch erhalten, daß man eine Halogenmagnesiumverbindung der Formel VI mit einem Nitril der Formel VII umsetzt und das erhaltene Produkt anschließend hydrolysiert.

Verbindungen der Formeln VI und VII sind bekannt oder können nach an sich bekannten Methoden hergestellt werden.

Diese Umsetzungen erfolgen vorzugsweise unter Bedingungen wie sie für Grignard-Reaktionen oder andere organometallischen Reaktionen bekannt sind, zweckmäßig in Gegenwart oder Abwesenheit eines inerten Lösungsmittels bei Temperaturen zwischen etwa -20 und etwa + 150 _{°} , vorzugsweise zwischen 0 und 150_{°}. Als Lösungsmittel eignen sich die oben angegebenen, soweit sie nicht selbst mit den Verbindungen der Formel VI reagieren können.

Weiterhin kann man eine Verbindung der Formel 1 auch dadurch erhalten, indem man eine Verbindung der Formel VIII mit einem Alkyl- oder Acylderivat der Formel IX umsetzt.

Verbindungen der Formel VIII sind bekannt und können in Analogie zu den in DE 40 41 074 A1 beschriebenen Verbindungen hergestellt werden.

Die Alkyl- und Acylderivate der Formel IX sind ebenfalls bekannt. Diese Verbindungen können nach einfachen organisch-chemischen Synthesevorschriften wie sie z.B. in J. March Adv. Org. Chem., 3'^{d} Ed., J. Wiley & Sons New York (1985) beschrieben sind, hergestellt werden.

Die Umsetzungen der beiden Verbindungen erfolgen vorzugsweise unter Bedingungen wie sie für die Alkylierung und Acylierung von primären und sekundären Aminen bekannt sind. Es ist zweckmäßig die Reaktionen in Gegenwart oder Abwesenheit eines inerten Lösungsmittels bei Temperaturen zwischen etwa -10 und etwa + 180 °, vorzugsweise zwischen 0 und 150 _{°} durchzuführen. Als Lösungsmittel eignen sich die zuvor angegebenen, soweit diese nicht selbst an der Reaktion teilnehmen können.

Ebenso ist es möglich, einen oder beide Rest(e) R⁴ und/oder R⁵ in (einen) andere(n) Rest(e) R⁴ und/oder R⁵ umzuwandeln. Man kann z.B., unter Verwendung von an sich bekannten Reaktionen, eine N0₂-Gruppe zu einer NH₂-Gruppe reduzieren, eine NH₂- oder NHA-Gruppe alkylieren, eine OH-Gruppe verethern oder aber einen Arylether spalten. Desweiteren können Substituenten dieser Reste R⁴ und/oder R⁵, wie z.B. S-R⁶⁻ oder SO-R⁶⁻Gruppen oxidiert werden, sofern die Reaktionen selektiv an den Resten R⁴ und/oder R⁵ stattfinden.

Eine Base der Formel 1 kann mit einer Säure in das zugehörige Säureadditionssalz überführt werden. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-oder 3-Phenylpropionsäure, Citronensäure, Gluconsäure, Ascorbinsäure, Nicotinsäure, Isonicotinsäure, Methan- und Ethansulfonsäure, Ethandisulfonsäure, 2-Hydroxyethansulfonsäure, Benzolsulfonsäure, p-Toluolsulfonsäure, Naphthalin-mono- und disulfonsäuren, Laurylschwefelsäure. Salze mit physiologisch nicht unbedenklichen Säuren, z.B. Pikrate, können zur Aufreinigung der Verbindungen der Formel I verwendet werden.

Verbindungen der Formel I können ein oder mehrere Asymmetriezentren enthalten. In diesem Fall liegen sie gewöhnlich in racemischer Form vor. Erhaltene Racemate können nach an sich bekannten Methoden mechanisch oder chemisch in ihre optischen Antipoden getrennt werden. Vorzugsweise werden aus dem racemischen Gemisch durch Umsetzung mit einem optisch-aktiven Trennmittel Diastereomere gebildet. Als Trennmittel für basische Verbindungen der Formel I eignen sich z.B. optisch aktive Säuren, wie die D- und L-Formen von Weinsäure, Diacetylweinsäure, Dibenzoylweinsäure, Mandelsäure, Äpfelsäure, Milchsäure oder die verschiedenen optisch-aktiven Camphersulfonsäuren, wie β-Camphersulfonsäure oder aber optisch aktive Camphansäure bzw. andere optisch aktive Terpensäuren.

Natürlich ist es auch möglich, optisch aktive Verbindungen der Formel 1 nach den oben beschriebenen Methoden zu erhalten, indem man Ausgangsstoffe verwendet, die bereits optisch aktiv sind.

Darüber hinaus zeigen die Verbindungen der Formel I, in Analogie zur E-Z-Isomerie bei C = C-Doppelbindungen, eine vergleichbare Stereoisomerie an der nicht-cyclischen C = N-Ooppelbindung. Die Verbindungen können somit als Mischungen der möglichen Stereoisomeren oder aber als reine E- oder Z-Isomere vorliegen.

Gegenstand der Erfindung ist ferner die Verwendung der Verbindungen der Formel I und ihrer physiologischen unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielweise Wasser, pflanzliche Öle, Benzylalkohole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes, Sticks oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I können bei der Bekämpfung von Krankheiten, insbesondere von Arrhythmien und von Herzinsuffizienzen sowie bei der therapeutischen Behandlung des menschlichen oder tierischen Körpers verwendet werden.

Dabei werden die erfindungsgemäßen Substanzen in der Regel in Analogie zu bekannten positiv inotrop wirksamen Substanzen wie Amrinon verabreicht, vorzugsweise in Dosierungen zwischen etwa 1 und 100 mg, insbesondere zwischen 2 und 20 mg pro Dosierungseinheit.

Die tägliche Dosierung liegt vorzugsweise zwischen etwa 0,02 und 2 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt. Im Vergleich zu den bisher zur Therapie der Herzinsuffizienz verwendeten Digitalis-Glykosiden zeichnen sich die Verbindungen der Formel I durch verbesserte therapeutische Breite und periphere Entlastung aus.

In den nachfolgenden Beispielen bedeutet "übliche Aufarbeitung":
Man gibt, falls erforderlich, Wasser oder verdünnte Natronlauge hinzu, extrahiert mit einem organischen Lösungsmittel wie Ethylacetat, Chloroform oder Dichlormethan, trennt ab, trocknet die organische Phase über Natriumsulfat, filtriert, dampft ein und reinigt durch Chromatographie und/oder Kristallisation.

Vor- und nachstehend sind alle Temperaturen in Celsiusgraden angegeben.

### Beispiel 1

Eine Lösung von 2,6 g 5-(1,2,3,4-Tetrahydrochinolin-6-yl)-6,6-dimethyl-3,6-dihydro-1,3,4-thiadiazin-2-on ("A") [erhältlich aus Hydrazinthioameisensäure-O-ethylester und 6-(2-Chlor-2-methyl-propionyl)-2-oxo-1,2,3,4-tetrahydrochinolin] in 40 ml Dichlormethan und 1 ml Pyridin wird mit 2,1 g N-Ethyl-3,4-dimethoxy- benzimidoylchlorid [erhältlich aus N-Ethyl-3,4-dimethoxybenzamid durch Umsetzung mit Thionylchlorid] gelöst in 20 ml Dichlormethan unter Eiskühlung versetzt und 1 Stunde gerührt. Nach Entfernung des Lösungsmittels arbeitet man wie üblich auf und erhält 5-[1-(N-Ethyl-3,4-dimethoxybenzimidoyl)-1,2,3,4-tetrahydrochinolin-6-yl]-6,6-dimethyl-3,6-dihydro-2H-1,3,4-thiadiazin-2-on, F. 188 °.

Analog erhält man:
ausgehend von "A" durch Umsetzung
mit N-Ethyl-p-methoxy-benzimidoylchlorid;
5- [1- (N-Ethyl-p-methoxybenzimidoyl)-1,2,3,4-tetrahydrochinolin-6-yl]-6,6-dimethyl-3,6-dihydro-2H-1,3,4-thiadiazin-2-on, F. 244° (Hydrochlorid);
mit N-Phenyl-p-methoxy-benzimidoylchlorid:
   5-[1-(N-Phenyl-p-methoxybenzimidoyl)-1,2,3,4-tetrahydrochinolin-6-yl]-6,6-dimethyl-3,6-dihydro-2H-1,3,4-thiadiazin-2-on, F. 210 °(Hydrochlorid);
mit N-Ethyl-p-chlor-benzimidoylchlorid:
   5-[1-(N-Ethyl-p-chlorbenzimidoyl)-1,2,3,4-tetrahydrochinolin-6-yl]-6,6-dimethyl-3,6-dihydro-2H-1,3,4-thiadiazin-2-on, F. 254° (Hydrochlorid);
mit N-Ethyl-2-thienylimidoylchlorid:
   5-[1-(N-Ethyl-2-thienyl-imidoyl)-1,2,3,4-tetrahydrochinolin-6-yl]-6,6-dimethyl-3,6-dihydro-2H-1,3,4-thiadiazin-2-on, F. 223 °(Hydrochlorid);
mit N-(2-Phenyl-ethyl)-p-methoxy-benzimidoylchlorid:
   5-[1-(N-(2-Phenyl-ethyl-p-methoxybenzimidoyl)-1,2,3,4-tetrahydrochinolin-6-yl]-6,6-dimethyl-3,6-dihydro-2H-1,3,4-thiadiazin-2-on, F. 245 ° (Hydrochlorid)
mit N-Ethyl-o-methoxy-benzimidoylchlorid;
5-[1-(N-Ethyl-o-methoxybenzimidoyl)-1,2,3,4-tetrahydrochinolin-6-yl]-6,6-dimethyl-3,6-dihydro-2H-1,3,4-thiadiazin-2-on;
mit N-Ethyl-m-methoxy-benzimidoylchlorid;
5-[1-(N-Ethyl-m-methoxybenzimidoyl)-1,2,3,4-tetrahydrochinolin-6-yl]-6,6-dimethyl-3,6-dihydro-2H-1,3,4-thiadiazin-2-on;
mit N-Ethyl-2,4-dimethoxy-benzimidoylchlorid:
   5-[1-(N-Ethyl-2,4-dimethoxybenzimidoyl)-1,2,3,4-tetrahydrochinolin-6-yl]-6,6-dimethyl-3,6-dihydro-2H-1,3,4-thiadiazin-2-on;
mit N-Methyl-p-methoxy-benzimidoylchlorid:
   5-[1-(N-Methyl-p-methoxybenzimidoyl)-1,2,3,4-tetrahydrochinolin-6-yl]-6,6-dimethyl-3,6-dihydro-2H-1,3,4-thiadiazin-2-on;
mit N-Ethyl-3,4-dichlor-benzimidoylchlorid:
   5-[1-(N-Ethyl-3,4-dichlorbenzimidoyl)-1,2,3,4-tetrahydrochinolin-6-yl]-6,6-dimethyl-3,6-dihydro-2H-1,3,4-thiadiazin-2-on;
mit N-Ethyl-2,4-dichlor-benzimidoylchlorid:
   5-[1-[N-Ethyl-2,4-dichlor-benzimidoyl)-1,2,3,4-tetrahydrochinolin-6yl]-6,6-dimethyl-3,6-dihydro-2H-1,3,4-thiadiazin-2-on;
mit N-Isopropyl-p-methoxy-benzimidoylchlorid:
   5-[1-(N-Isopropyl-p-methoxybenzimidoyl)-1,2,3,4-tetrahydrochinolin-6-yl]-6,6-dimethyl-3,6-dihydro-2H-1,3,4-thiadiazin-2-on;
mit N-Cyclohexyl-p-methoxy-benzimidoylchlorid:
   5-[1-(N-Cyclohexyl-p-methoxybenzimidoyl)-1,2,3,4-tetrahydrochinolin-6-yl]-6,6-dimethyl-3,6-dihydro-2H-1,3,4-thiadiazin-2-on;
mit N-Isopropyl-p-chlor-benzimidoylchlorid:
   5-[1-(N-Isopropyl-p-chlorbenzimidoyl)-1,2,3,4-tetrahydrochinolin-6-yl]-6,6-dimethyl-3,6-dihydro-2H-1,3,4-thiadiazin-2-on;
mit N-lsopropyl-2-thienylimidoylchlorid:
   5-[1-(N-Isopropyl-2-thienylimidoyl)-1,2,3,4-tetrahydrochinolin-6-yl]-6,6-dimethyl-3,6-dihydro-2H-1,3,4-thiadiazin-2-on;
mit N-Benzyl-p-methoxy-benzimidoylchlorid:
   5-[1-(N-Benzyl-p-methoxybenzimidoyl)-1,2,3,4-tetrahydrochinolin-6-yl]-6,6-dimethyl-3,6-dihydro-2H-1,3,4-thiadiazin-2-on;
mit N-Isopropyl-o-methoxy-benzimidoylchlorid:
   5-[1-(N-Isopropyl-o-methoxybenzimidoyl)-1,2,3,4-tetrahydrochinolin-6-yl]-6,6-dimethyl-3,6-dihydro-2H-1,3,4-thiadiazin-2-on;
mit N-Isopropyl-m-methoxy-benzimidoylchlorid:
   5-[1-(N-Isopropyl-m-methoxybenzimidoyl)-1,2,3,4-tetrahydrochinolin-6-yl]-6,6-dimethyl-3,6-dihydro-2H-1,3,4-thiadiazin-2-on;
mit N-Isopropyl-2,4-dimethoxy-benzimidoylchlorid:
   5-[1-(N-Isopropyl-2,4-dimethoxybenzimidoyl)-1,2,3,4-tetrahydrochinolin-6-yl]-6,6-dimethyl-3,6-dihydro-2H-1,3,4-thiadiazin-2-on;
mit N-Cyclohexyl-p-methoxy-benzimidoylchlorid:
   5-[1-(N-Cyclohexyl-p-methoxybenzimidoyl)-1,2,3,4-tetrahydrochinolin-6-yl]-6,6-dimethyl-3,6-dihydro-2H-1,3,4-thiadiazin-2-on;
mit N-Isopropyl-3,4-dichlor-benzimidoylchlorid:
   5-[1-(N-Isopropyl-3,4-dichlorbenzimidoyl)-1,2,3,4-tetrahydrochinolin-6-yl]-6,6-dimethyl-3,6-dihydro-2H-1,3,4-thiadiazin-2-on;
mit N-Isopropyl-2,4-dichlor-benzimidoylchlorid:
   5-[1-(N-Isopropyl-2,4-dichlor-benzimidoyl)-1,2,3,4-tetrahydrochinolin-6-yl]-6,6-dimethyl-3,6-dihydro-2H-1,3,4-thiadiazin-2-on.

### Beispiel 2

Analog Beispiel 1 erhält man ausgehend von "A" durch Umsetzung mit N-Hydroxy-3,4-dimethoxyben- zoesäureimidchlorid das 5-[1-N-Hydroxy-3,4-dimethoxybenzimidoyl)-1,2,3,4-tetrahydrochinolin-6yl]-6,6-dimethyl-3,6-dihydro-2H-1,3,4-thiadiazin-2-on.

Analog erhält man:
ausgehend von "A" durch Umsetzung
mit N-Hydroxy-p-methoxybenzoesäureimidchlorid:
   5-[1-(N-Hydroxy-p-methoxybenzimidoyl)-1,2,3,4-tetrahydrochinolin-6-yl]-6,6-dimethyl-3,6-dihydro-2H-1,3,4-thiadiazin-2-on;
mit N-Hydroxy-p-fluor-benzoesäureimidchlori:
   5-[1-(N-Hydroxy-p-fluorbenzimidoyl)-1,2,3,4-tetrahydrochinolin-6-yl]-6,6-dimethyl-3,6-dihydro-2H-1,3,4-thiadiazin-2-on;
mit N-Hydroxy-p-chlor-benzoesäureimidchlorid:
   5-[1-(N-Hydroxy-p-chlorbenzimidoyl)-1,2,3,4-tetrahydrochinolin-6-yl]-6,6-dimethyl-3,6-dihydro-2H-1,3,4-thiadiazin-2-on;
mit N-Hydroxy-p-methylthio-benzoesäureimidchlorid:
   5-[1-(N-Hydroxy-p-methylthio-benzimidoyl)-1,2,3,4-tetrahydrochinolin-6-yl]-6,6-dimethyl-3,6-dihydro-2H-1,3,4-thiadiazin-2-on;
mit N-Hydroxy-o-methoxy-benzoesäureimidchlorid:
   5-[1-(N-Hydroxy-o-methoxy-benzimidoyl)-1,2,3,4-tetrahydrochinolin-6-yl]-6,6-dimethyl-3,6-dihydro-2H-1,3,4-thiadiazin-2-on;
mit N-Hydroxy-m-methoxy-benzoesäureimidchlorid:
   5-[1-(N-Hydroxy-m-methoxy-benzimidoyl)-1,2,3,4-tetrahydrochinolin-6-yl]-6,6-dimethyl-3,6-dihydro-2H-1,3,4-thiadiazin-2-on;
mit N-Hydroxy-2,4-dimethoxy-benzoesäureimidchlorid:
   5-[1-(N-Hydroxy-2,4-dimethoxybenzimidoyl)-1,2,3,4-tetrahydrochinolin-6-yl]-6,6-dimethyl-3,6-dihydro-2H-1,3,4-thiadiazin-2-on;
mit N-Hydroxy-p-methylsulfonyl-benzoesäureimidchlorid:
   5-[1-(N-Hydroxy-p-methylsulfonyl-benzimidoyl)-1,2,3,4-tetrahydrochinolin-6-yl]-6,6-dimethyl-3,6-dihydro-2H-1,3,4-thiadiazin-2-on;
mit N-Hydroxy-3,4-dichlor-benzoesäureimidchlorid:
   5-[1-(N-Hydroxy-3,4-dichlorbenzimidoyl)-1,2,3,4-tetrahydrochinolin-6-yl]-6,6-dimethyl-3,6-dihydro-2H-1,3,4-thiadiazin-2-on;
mit N-Hydroxy-2,4-dichlor-benzoesäureimidchlorid:
   5-[1-(N-Hydroxy-2,4-dichlor-benzimidoyl)-1,2,3,4-tetrahydrochinolin-6-yl]-6,6-dimethyl-3,6-dihydro-2H-1,3,4-thiadiazin-2-on;
mit N-Hydroxy-p-ethylthio-benzoesäureimidchlorid:
   5-[1-(N-Hydroxy-p-ethylthio-benzimidoyl)-1,2,3,4-tetrahydrochinolin-6-yl]-6,6-dimethyl-3,6-dihydro-2H-1,3,4-thiadiazin-2-on;
mit N-Hydroxy-p-methylsulfinyl-benzoesäureimidchlorid:
   5-[1-(N-Hydroxy-p-methylsulfinyl-benzimidoyl)-1,2,3,4-tetrahydrochinolin-6-yl]-6,6-dimethyl-3,6-dihydro-2H-1,3,4-thiadiazin-2-on;
mit N-Hydroxy-p-nitro-benzoesäureimidchlorid:
   5-[1-(N-Hydroxy-p-nitrobenzimidoyl)-1,2,3,4-tetrahydrochinolin-6-yl]-6,6-dimethyl-3,6-dihydro-2H-1,3,4-thiadiazin-2-on;
mit N-Hydroxy-(p-N-di-methylamino)-benzoesäure imidchlorid:
   5-[1-(N-Hydroxy-(p-N-di-methylamino)-benzimidoyl)-1,2,3,4-tetrahydrochinolin-6-yl]-6,6-dimethyl-3,6-dihydro-2H-1,3,4-thiadiazin-2-on;
mit N-Hydroxy-p-acetamido-benzoesäureimidchlorid:
   5-[1-(N-Hydroxy-p-acetamidobenzimidoyl)-1,2,3,4-tetrahydrochinolin-6-yl]-6,6-dimethyl-3,6-dihydro-2H-1,3,4-thiadiazin-2-on;
mit N-Hydroxy-p-methansulfonamido-benzoesäureimidchlorid:
   5-[1-(N-Hydroxy-p-methansulfonamido-benzimidoyl)-1,2,3,4-tetrahydrochinolin-6-yl]-6,6-dimethyl-3,6-dihydro-2H-1,3,4-thiadiazin-2-on;
mit N-Hydroxy-3,4-dinitro-benzoesäureimidchlorid:
   5-[1-(N-Hydroxy-3,4-dinitrobenzimidoyl)-1,2,3,4-tetrahydrochinolin-6-yl]-6,6-dimethyl-3,6-dihydro-2H-1,3,4-thiadiazin-2-on.

### Beispiel 3

Zu einer Lösung von 3,4 g 5-[1-N-Hydroxy-3,4-dimethoxybenzimidoyl)-1,2,3,4-tetrahydrochinolin-6-yl]-6,6-dimethyl-3,6-dihydro-2H-1,3,4-thiadiazin-2-on in 30 ml Tetrahydrofuran wird 1 Äquivalent Eisenpentacarbonyl zugetropft. Die Reaktionsmischung wird 2 Stunden gekocht und wie üblich aufgearbeitet. Man erhält 5-[1-(3,4-Dimethoxybenzimidoyl)-1,2,3,4-tetrahydrochinolin-6-yl]-6,6-dimethyl-3,6-dihydro-2H-1,3,4-thiadiazin-2-on.

Analog erhält man aus den entsprechenden N-Hydroxybenzimidoyl-Derivaten:
5-[1-(p-Methoxybenzimidoyl)-1,2,3,4-tetrahydrochinolin-6-yl]-6,6-dimethyl-3,6-dihydro-2H-1,3,4-thiadiazin-2- on;
5-[1-(p-Fluorbenzimidoyl)-1,2,3,4-tetrahydrochinolin-6-yl]-6,6-dimethyl-3,6-dihydro-2H-1,3,4-thiadiazin-2-on;
5-[1-(p-Chlorbenzimidoyl)-1,2,3,4-tetrahydrochinolin-6-yl]-6,6-dimethyl-3,6-dihydro-2H-1,3,4-thiadiazin-2-on;
5-[1-(p-Methylthio-benzimidoyl)-1,2,3,4-tetrahydrochinolin-6-yl]-6,6-dimethyl-3,6-dihydro-2H-1,3,4-thiadiazin-2-on;
5-[1-(o-Methoxybenzimidoyl)-1,2,3,4-tetrahydrochinolin-6-yl]-6,6-dimethyl-3,6-dihydro-2H-1,3,4-thiadiazin-2- on;
5-[1-(m-Methoxybenzimidoyl)-1,2,3,4-tetrahydrochinolin-6-yl]-6,6-dimethyl-3,6-dihydro-2H-1,3,4-thiadiazin-2- on;
5-[1-(2,4-Dimethoxybenzimidoyl)-1,2,3,4-tetrahydrochinolin-6-yl]-6,6-dimethyl-3,6-dihydro-2H-1,3,4-thiadiazin-2-on;
5-[1-(p-Methylsulfonyl-benzimidoyl)-1,2,3,4-tetrahydrochinolin-6-yl]-6,6-dimethyl-3,6-dihydro-2H-1,3,4-thiadiazin-2-on;
5-[1-(3,4-Dichlorbenzimidoyl)-1,2,3,4-tetranydrochinolin-6-yl]-6,6-dimethyl-3,6-dihydro-2H-1,3,4-thiadiazin-2- on;
5-[1-(2,4-Dichlor-benzimidoyl)-1,2,3,4-tetrahydrochinolin-6-yl]-6, 6-dimethyl-3, 6-dihydro-2H-1,3,4-thiadiazin-2-on;
5-[1-(p-Ethylthiobenzimidoyl)-1,2,3,4-tetrahydrochinolin-6-yl]-6,6-dimethyl-3,6-dihydro-2H-1,3,4-thiadiazin-2- on;
5-[1-(p-Methylsulfinyl-benzimidoyl)-1,2,3,4-tetrahydrochinolin-6-yl]-6,6-dimethyl-3,6-dihydro-2H-1,3,4-thiadiazin-2-on;
5-[1-(p-Nitrobenzimidoyl)-1,2,3,4-tetrahydrochinolin-6-yl]-6,6-dimethyl-3,6-dihydro-2H-1,3,4-thiadiazin-2-on;
5-[1-(p-(N,N-Di-methylamino-benzimidoyl)-1,2,3,4-tetrahydrochinolin-6-yl]-6,6-dimethyl-3,6-dihydro-2H-1,3,4-thiadiazin-2-on;
5-[1-(p-Acetamido-benzimidoyl)-1,2,3,4-tetrahydrochinolin-6-yl]-6,6-dimethyl-3,6-dihydro-2H-1,3,4-thiadiazin-2-on;
5-[1-(o-Acetamido-benzimidoyl)-1,2,3,4-tetrahydrochinolin-6-yl]-6,6-dimethyl-3,6-dihydro-2H-1,3,4-thiadiazin-2-on;
5-[1-(p-Methansulfonamido-benzimidoyl)-1,2,3,4-tetrahydrochinolin-6-yl]-6,6-dimethyl-3,6-dihydro-2H-1,3,4-thiadiazin-2-on;
5-[1-(3,4-Dinitrobenzimidoyl)-1,2,3,4-tetrahydrochinolin-6-yl]-6,6-dimethyl-3,6-dihydro-2H-1,3,4-thiadiazin-2- on.

### Beispiel 4

Eine Lösung von 1,7 g 1-(N-Ethyl-p-methoxybenzimidoyl)-6-(2-chlor-2-methyl-propionyl)-1,2,3,4-tetrah- ydrochinolin in 40 ml Acetonitril wird mit 1 Äquivalent Hydrazinthioameisensäure-O-ethylester versetzt und 2 Stunden gekocht. Nach üblicher Aufarbeitung erhält man 5-[1-(N-Ethyl-p-methoxybenzimidoyl)-1,2,3,4-tetrahydrochinolin-6-yl]-6,6-dimethyl-3,6-dihydro-2H-1,3,4-thiadiazin-2-on, F. 244 °(Hydrochlorid).

Analog erhält man durch Umsetzung von Hydrazinthioameisensäure-O-ethylester
mit 1-(N-Benzyl-p-methoxybenzimidoyl)-6-(2-chlor-2-methyl-propionyl)-1,2,3,4-tetrahydrochinolin:
   5-[1-N-Benzyl-p-methoxybenzimidoyl)-1,2,3,4-tetrahydrochinolin-6-yl]-6,6-dimethyl-3,6-dihydro-2H-1,3,4-thiadiazin-2-on;
mit 1-(N-Cyclopentyl-p-methoxybenzimidoyl)-6-(2-chlor-2-methyl-propionyl)-1,2,3,4-tetrahydrochinolin:
   5-[1-N-Cyclopentyl-p-methoxybenzimidoyl)-1,2,3,4-tetrahydrochinolin-6-yl]-6,6-dimethyl-3,6-dihydro-2H-1,3,4-thiadiazin-2-on;
mit 1-(N-Cyclohexyl-p-methoxybenzimidoyl)-6-(2-chlor-2-methyl-propionyl)-1,2,3,4-tetrahydrochinolin:
   5-[1-N-Cyclohexyl-p-methoxybenzimidoyl)-1,2,3,4-tetrahydrochinolin-6-yl]-6,6-dimethyl-3,6-dihydro-2H-1,3,4-thiadiazin-2-on;
mit 1-(N-(2-Phenyl-ethyl)-p-methoxybenzimidoyl)-6-(2-chlor-2-methyl-propionyl)-1,2,3,4-tetrahydrochinolin:
   5-[1-N-(2-Phenyl-ethyl)-p-methoxybenzimidoyl)-1,2,3,4-tetrahydrochinolin-6-yl]-6,6-dimethyl-3,6-dihydro-2H-1,3,4-thiadiazin-2-on;
mit 1-(N-(2-Phenyl-ethyl)-3,4-dimethoxybenzimidoyl)-6-(2-chlor-2-methyl-propionyl)-1,2,3,4-tetrahydrochino- lin:
   5-[1-N-(2-Phenyl-ethyl)-3,4-dimethoxybenzimidoyl)-1,2,3,4-tetrahydrochinolin-6-yl]-6,6-dimethyl-3,6-dihydro-2H-1,3,4-thiadiazin-2-on.

### Beispiel 5

Eine Lösung von 1,2 g 6-(6,6-Dimethyl-2-oxo-3,6-dihydro-2H-1,3,4-thiadiazin-5-yl)-1,2,3,4-tetrahydrochinolin-1-magnesiumbromid ("B") in 40 ml Tetrahydrofuran wird tropfenweise mit einem Äquivalent p-Chlorbenzonitril versetzt und 1,5 Stunden gekocht. Man erhält nach üblicher Aufarbeitung 5-[1-(p-Chlorbenzimidoyl)-1,2,3,4-tetrahydrochinolin-6-yl]-6,6-dimethyl-3,6-dihydro-2H-1,3,4-thiadiazin-2-on.

### Beispiel 6

Eine Lösung von 2,8 g 5-[1-N-Ethyl-3,4-dimethoxybenzimidoyl)-1,2,3,4-tetrahydrochinolin-6-yl]-6,6-dimethyl-3,6-dihydro-2H-1,3,4-thiadiazin-2-on (F. 188_{°}) in 40 ml Dimethylformamid wird unter Eiskühlung mit 0,3 g NaH versetzt und 1 Stunde gerührt. Anschließend fügt man 1,5 ml Ethyliodid hinzu, rührt weitere 2 Stunden und erhält nach üblicher Aufarbeitung 5-[1-N-Ethyl-3,4-dimethoxyhenzimidoyl)-1,2,3,4-tetrahydrochinolin-6-yl]-3-ethyl-6,6-dimethyl-3,6-dihydro-2H-1,3,4-thiadiazin-2-on.

Analog erhält man durch Alkylierung der entsprechenden Verbindungen der Formel I in 3-Position des Thiadiazinon-Systems:
5-[1-(N-Ethyl-p-methoxybenzimidoyl)-1,2,3,4-tetrahydrochinolin-6-yl]-3-ethyl-6,6-dimethyl-3,6-dihydro-2H-1,3,4-thiadiazin-2-on;
5-[1-(N-Phenyl-p-methoxybenzimidoyl)-1,2,3,4-tetrahydrochinolin-6-yl]-3-ethyl-6,6-dimethyl-3,6-dihydro-2H-1,3,4-thiadiazin-2-on;
5-[1-(N-Ethyl-p-chlorbenzimidoyl)-1,2,3,4-tetrahydrochinolin-6-yl]-3-ethyl-6,6-dimethyl-3,6-dihydro-2H-1,3,4-thiadiazin-2-on;
5-[1-(N-Ethyl-2-thienylimidoyl)-1,2,3,4-tetrahydrochinolin-6-yl]-3-ethyl-6,6-dimethyl-3,6-dihydro-2H-1,3,4-thiadiazin-2-on;
5-[1-(N-(2-Phenyl-ethyl)-p-methoxybenzimidoyl)-1,2,3,4-tetrahydrochinolin-6-yl]-3-ethyl-6,6-dimethyl-3,6-dihydro-2H-1,3,4-thiadiazin-2-on;
5-[1-(N-Ethyl-o-methoxybenzimidoyl)-1,2,3,4-tetrahydrochinolin-6-yl]-3-ethyl-6,6-dimethyl-3,6-dihydro-2H-1,3,4-thiadiazin-2-on;
5-[1-(N-Ethyl-m-methoxybenzimidoyl)-1,2,3,4-tetrahydrochinolin-6-yl]-3-ethyl-6,6-dimethyl-3,6-dihydro-2H-1,3,4-thiadiazin-2-on;
5-[1-(N-Ethyl-2,4-dimethoxybenzimidoyl)-1,2,3,4-tetrahydrochinolin-6-yl]-3-ethyl-6,6-dimethyl-3,6-dihydro-2H-1,3,4-thiadiazin-2-on;
5-[1-(N-Methyl-p-methoxybenzimidoyl)-1,2,3,4-tetrahydrochinolin-6-yl]-3-ethyl-6,6-dimethyl-3,6-dihydro-2H-1,3,4-thiadiazin-2-on;
5-[1-(N-Ethyl-3,4-dichlorbenzimidoyl)-1,2,3,4-tetrahydrochinolin-6-yl]-3-ethyl-6,6-dimethyl-3,6-dihydro-2H-1,3,4-thiadiazin-2-on;
5-[1-(N-Ethyl-2,4-dichlor-benzimidoyl)-1,2,3,4-tetrahydrochinolin-6-yl]-3-ethyl-6,6-dimethyl-3,6-dihydro-2H-1,3,4-thiadiazin-2-on;
5-[1-(N-Isopropyl-p-methoxybenzimidoyl)-1,2,3,4-tetrahydrochinolin-6-yl]-3-ethyl-6,6-dimethyl-3,6-dihydro-2H-1,3,4-thiadiazin-2-on;
5-[1-(N-Cyclohexyl-p-methoxyhenzimidoyl)-1,2,3,4-tetrahydrochinolin-6-yl]-3-ethyl-6,6-dimethyl-3,6-dihydro-2H-1,3,4-thiadiazin-2-on;
5-[1-(N-Isopropyl-p-chlor-benzimidoyl)-1,2,3,4-tetrahydrochinolin-6-yl]-3-ethyl-6,6-dimethyl-3,6-dihydro-2H-1,3,4-thiadiazin-2-on;
5-[1-(N-Isopropyl-2-thienylimidoyl)-1,2,3,4-tetrahydrochinolin-6-yl]-3-ethyl-6,6-dimethyl-3,6-dihydro-2H-1,3,4-thiadiazin-2-on;
5-[1-(N-Benzyl-p-methoxybenzimidoyl)-1,2,3,4-tetrahydrochinolin-6-yl]-3-ethyl-6,6-dimethyl-3,6-dihydro-2H-1,3,4-thiadiazin-2-on;
5-[1-(N-Isopropyl-o-methoxybenzimidoyl)-1,2,3,4-tetrahydrochinolin-6-yl]-3-ethyl-6,6-dimethyl-3,6-dihydro-2H-1,3,4-thiadiazin-2-on;
5-[1-(N-Isopropyl-m-methoxybenzimidoyl)-1,2,3,4-tetrahydrochinolin-6-yl]-3-ethyl-6,6-dimethyl-3,6-dihydro-2H-1,3,4-thiadiazin-2-on;
5-[1-(N-Isopropyl-2,4-dimethoxybenzimidoyl)-1,2,3,4-tetrahydrochinolin-6-yl]-3-ethyl-6,6-dimethyl-3,6-dihydro-2H-1,3,4-thiadiazin-2-on;
5-[1-(N-Cyclohexyl-p-methoxybenzimidoyl)-1,2,3,4-tetrahydrochinolin-6-yl]-3-ethyl-6,6-dimethyl-3,6-dihydro-2H-1,3,4-thiadiazin-2-on;
5-[1-(N-Isopropyl-3,4-dichlorbenzimidoyl)-1,2,3,4-tetrahydrochinolin-6-yl]-3-ethyl-6,6-dimethyl-3,6-dihydro-2H-1,3,4-thiadiazin-2-on;
5-[1-(N-Isopropyl-2,4-dichlor-benzimidoyl)-1,2,3,4-tetrahydrochinolin-6-yl]-3-ethyl-6,6-dimethyl-3,6-dihydro-2H-1,3,4-thiadiazin-2-on;
5-[1-(N-Ethyl-p-methoxybenzimidoyl)-1,2,3,4-tetrahydrochinolin-6-yl]-3,6,6-trimethyl-6,6-dimethyl-3,6-dihydro2H-1,3,4-thiadiazin-2-on;
5-[1-(N-Phenyl-p-methoxybenzimidoyl)-1,2,3,4-tetrahydrochinolin-6-yl]-3,6,6-trimethyl-3,6-dihydro-2H-1,3,4-thiadiazin-2-on;
5-[1-(N-Ethyl-p-nitrobenzimidoyl)-1,2,3,4-tetrahydrochinolin-6-yl]-3,6,6-trimethyl-3,6-dihydro-2H-1,3,4-thiadiazin-2-on;
5-[1-(N-Ethyl-2-thienylimidoyl)-1,2,3,4-tetrahydrochinolin-6-yl]-3,6,6-trimethyl-3,6-dihydro-2H-1,3,4-thiadiazin-2-on;
5-[1-(N-(2-Phenyl-ethyl)-p-methoxybenzimidoyl)-1,2,3,4-tetrahydrochinolin-6-yl]-3,6,6-trimethyl-6,6-dimethyl-3,6-dihydro-2H-1,3,4-thiadiazin-2-on;
5-[1-(N-Ethyl-o-methoxybenzimidoyl)1,2,3,4-tetrahydrochinolin-6-yl]-3,6,6-trimethyl-3,6-dihydro-2H-1,3,4-thiadiazin-2-on;
5-[1-(N-Ethyl-m-methoxybenzimidoyl)-1,2,3,4-tetrahydrochinolin-6-yl]-3,6,6-trimethyl-3,6-dihydro-2H-1,3,4-thiadiazin-2-on;
5-[1-(N-Ethyl-3,4-dimethoxybenzimidoyl)-1,2,3,4-tetrahydrochinolin-6yl]-3,6,6-trimethyl-3,6-dihydro-2H-1,3,4-thiadiazin-2-on;
5-[1-(N-Methyl-p-methoxybenzimidoyl)-1,2,3,4-tetrahydrochinolin-6-yl]-3,6,6-trimethyl-3,6-dihydro-2H-1,3,4-thiadiazin-2-on;
5-[1-(N-Ethyl-3,4-dichlor-benzimidoyl)-1,2,3,4-tetrahydrochinolin-6-yl]-3,6,6-trimethyl-3,6-dihydro-2H-1,3,4-thiadiazin-2-on;
5-[1-(N-Isopropyl-2,4-dichlor-benzimidoyl)-1,2,3,4-tetrahydrochinolin-6-yl]-3,6,6-trimethyl-3,6-dihydro-2H-1,3,4-thiadiazin-2-on;
5-[1-(N-Cyclohexyl-p-nitro-benzimidoyl)-1,2,3,4-tetrahydrochinolin-6-yl]-3,6,6-trimethyl-3,6-dihydro-2H-1,3,4-thiadiazin-2-on;
5-[1-(N-Isopropyl-p-chlorbenzimidoyl)-1,2,3,4-tetrahydrochinolin-6-yl]-3,6,6-trimethyl-3,6-dihydro-2H-1,3,4-thiadiazin-2-on;
5-[1-(N-Isopropyl-2-thienylimidoyl)-1,2,3,4-tetrahydrochinolin-6-yl]-3,6,6-trimethyl-3,6-dihydro-2H-1,3,4-thiadiazin-2-on;
5-[1-(N-Benzyl-p-methoxybenzimidoyl)-1,2,3,4-tetrahydrochinolin-6-yl]-3,6,6-trimethyl-3,6-dihydro-2H-1,3,4-thiadiazin-2-on;
5-[1-(N-Isopropyl-o-methoxybenzimidoyl)-1,2,3,4-tetrahydrochinolin-6-yl]-3,6,6-trimethyl-3,6-dihydro-2H-1,3,4-thiadiazin-2-on;
5-[1-(N-Isopropyl-m-methoxybenzimidoyl)-1,2,3,4-tetrahydrochinolin-6-yl]-3,6,6-trimethyl-3,6-dihydro-2H-1,3,4-thiadiazin-2-on;
5-[1-(N-Isopropyl-2,4-dimethoxybenzimidoyl)-1,2,3,4-tetrahydrochinolin-6-yl)-3,6,6-trimethyl-3,6-dihydro-2H-1,3,4-thiadiazin-2-on;
5-[1-(N-Cyclohexyl-p-methoxybenzimidoyl)-1,2,3,4-tetrahydrochinolin-6-yl]-3,6,6-trimethyl-3,6-dihydro-2H-1,3,4-thiadiazin-2-on;
5-[1-(N-Isopropyl-2,4-dichlorbenzimidoyl)-1,2,3,4-tetrahydrochinolin-6-yl]1-3,6,6-trimethyl-3,6-dihydro-2H-1,3,4-thiadiazin-2-on;
5-[1-(N-Methyl-3,4-dimethoxybenzimidoyl)-1,2,3,4-tetrahydrochinolin-6-yl]-3,6,6-trimethyl-3,6-dihydro-2H-1,3,4-thiadiazin-2-on;
5-[1-(N-Methyl-p-nitrobenzimidoyl)-1,2,3,4-tetrahydrochinolin-6-yl]-3,6,6-trimethyl-3,6-dihydro-2H-1,3,4-thiadiazin-2-on.

### Beispiel 7

Eine Lösung von 2,5 g 5-[1-N-Ethyl-3,4-dimethoxybenzimidoyl)-1,2,3,4-tetrahydrochinolin-6-yl]-6,6-dimethyl-3,6-dihydro-2H-1,3,4-thiadiazin-2-on (F. 188°) in 30 ml Dichlormethan wird mit 0,5 ml Triethylamin und anschließend tropfenweise unter Rühren mit 0,8 ml Acetylchlorid versetzt. Man rührt noch eine Stunde bei Raumtemperatur, zersetzt mit Wasser und erhält nach üblicher Aufarbeitung 5-[1-N-Ethyl-3,4-dimethoxy- benzimidoyl)-1,2,3,4-tetrahydrochinolin-6-yl]-3-acetyl-6,6-dimethyl-3,6-dihydro-2H-1,3,4-thiadiazin-2-on.

Analog erhält man durch Acylierung der entsprechenden Verbindungen der Formel I in 3-Position des Thiadiazinon-Systems (Formylchlorid wird dabei durch Einleiten von CO- und HCI-Gas, in Gegenwart von AlCl₃ und CuCl, in die Reaktionslösung erzeugt):
5-[1-(N-Ethyl-p-methoxybenzimidoyl)-1,2,3,4-tetrahydrochinolin-6-yl]-3-acetyl-6,6-dimethyl-3,6-dihydro-2H-1,3,4-thiadiazin-2-on;
5-[1-(N-Phenyl-p-methoxybenzimidoyl)-1,2,3,4-tetrahydrochinolin-6-yl]-3-formyl-6,6-dimethyl-3,6-dihydro-2H-1,3,4-thiadiazin-2-on;
5-[1-(N-Ethyl-p-chlorbenzimidoyl)-1,2,3,4-tetrahydrochinolin-6-yl]-3-acetyl-6,6-dimethyl-3,6-dihydro-2H-1,3,4-thiadiazin-2-on;
5-[1-(N-Ethyl-2-thienylimidoyl)-1,2,3,4-tetrahydrochinolin-6-yl]-3-acetyl-6,6-dimethyl-3,6-dihydro-2H-1,3,4-thiadiazin-2-on;
5-[1-(N-(2-Phenyl-ethyl-p-methoxybenzimidoyl)-1,2,3,4-tetrahydrochinolin-6-yl]-3-formyl-6,6-dimethyl-3,6-dihydro-2H-1,3,4-thiadiazin-2-on;
5-[1-(N-Ethyl-o-methoxybenzimidoyl)-1,2,3,4-tetrahydrochinolin-6-yl]-3-formyl-6,6-dimethyl-3,6-dihydro-2H-1,3,4-thiadiazin-2-on;
5-[1-(N-Ethyl-m-methoxybenzimidoyl)-1,2,3,4-tetrahydrochinolin-6-yl]-3-acetyl-6,6-dimethyl-3,6-dihydro-2H-1,3,4-thiadiazin-2-on;
5-[1-(N-Ethyl-2,4-dimethoxybenzimidoyl)-1,2,3,4-tetrahydrochinolin-6-yl]-3-acetyl-6,6-dimethyl-3,6-dihydro-2H-1,3,4-thiadiazin-2-on;
5-[1-(N-Methyl-p-methoxybenzimidoyl)-1,2,3,4-tetrahydrochinolin-6-yl]-3-acetyl-6,6-dimethyl-3,6-dihydro-2H-1,3,4-thiadiazin-2-on;
5-[1-(N-Ethyl-3,4-dichlorbenzimidoyl)-1,2,3,4-tetrahydrochinolin-6-yl]-3-acetyl-6,6-dimethyl-3,6-dihydro-2H-1,3,4-thiadiazin-2-on;
5-[1-(N-Ethyl-2,4-dichlorbenzimidoyl)-1,2,3,4-tetrahydrochinolin-6-yl]-3-formyl-6,6-dimethyl-3,6-dihydro-2H-1,3,4-thiadiazin-2-on;
5-[1-(N-Isopropyl-p-methoxybenzimidoyl)-1,2,3,4-tetrahydrochinolin-6yl]-3-acetyl-6,6-dimethyl-3,6-dihydro-2H-1,3,4-thiadiazin-2-on;
5-[1-(N-Isopropyl-p-chlorbenzimidoyl)-1,2,3,4-tetrahydrochinolin-6-yl]-3-acetyl-6,6-dimethyl-3,6-dihydro-2H-1,3,4-thiadiazin-2-on;
5-[1-(N-Isopropyl-2-thienylimidoyl)-1,2,3,4-tetrahydrochinolin-6-yl]-3-acety-6,6-dimethyl-3,6-dihydro-2H-1,3,4-thiadiazin-2-on;
5-[1-(N-Benzyl-p-methoxybenzimidoyl)-1,2,3,4-tetrahydrochinolin-6-yl]-3-acetyl-6,6-dimethyl-3,6-dihydro-2H-1,3,4-thiadiazin-2-on;
5-[1-(N-Isopropyl-o-methoxybenzimidoyl)-1,2,3,4-tetrahydrochinolin-6-yl]-3-formyl-6,6-dimethyl-3,6-dihydro-2H-1,3,4-thiadiazin-2-on;
5-[1-(N-Isopropyl-m-methoxybenzimidoyl)-1,2,3,4-tetrahydrochinolin-6-yl]-3-formyl-6,6-dimethyl-3,6-dihydro-2H-1,3,4-thiadiazin-2-on;
5-[1-(N-Isopropyl-2,4-dimethoxybenzimidoyl)-1,2,3,4-tetrahydrochinolin-6-yl]-3-formyl-6,6-dimethyl-3,6-dihydro-2H-1,3,4-thiadiazin-2-on;
5-[1-(N-Cylcohexyl-p-methoxybenzimidoyl)-1,2,3,4-tetrahydrochinolin-6-yl]-3-acetyl-6,6-dimethyl-3,6-dihydro-2H-1,3,4-thiadiazin-2-on;
5-[1-(N-Isopropyl-3,4-dichlorbenzimidoyl)-1,2,3,4-tetrahydrochinolin-6-yl]-3-acetyl-6,6-dimethyl-3,6-dihydro-2H-1,3,4-thiadiazin-2-on;
5-[1-(N-Isopropyl-2,4-dichlor-benzimidoyl)-1,2,3,4-tetrahydrochinolin-6-yl]-3-acetyl-6,6-dimethyl-3,6-dihydro-2H-1,3,4-thiadiazin-2-on;
5-[1-(N-Ethyl -p-methoxybenzimidoyl)-1,2,3,4-tetrahydrochinolin-6-yl]-3-butyryl-6,6-dimethyl-3,6-dihydro-2H-1,3,4-thiadiazin-2-on;
5-[1-(N-Phenyl-p-methoxybenzimidoyl)-1,2,3,4-tetrahydrochinolin-6-yl]-3-butyryl-6,6-dimethyl-3,6-dihydro-2H-1,3,4-thiadiazin-2-on;
5-[1-(N-Ethyl-p-chlorbenzimidoyl)-1,2,3,4-tetrahydrochinolin-6-yl]-3-propionyl-6,6-dimethyl-3,6-dihydro-2H-1,3,4-thiadiazin-2-on;
5-[1-(N-Ethyl-2-thienylimidoyl)-1,2,3,4-tetrahydrochinolin-6-yl]-3-butyryl-6,6-dimethyl-3,6-dihydro-2H-1,3,4-thiadiazin-2-on;
5-[1-(N-(2-Phenyl-ethyl-p-methoxybenzimidoyl)-1,2,3,4-tetrahydrochinolin-6-yl]-3-propionyl-6,6-dimethyl-3,6-dihydro-2H-1,3,4-thiadiazin-2-on;
5-[1-(N-Ethyl-o-methoxybenzimidoyl)-1,2,3,4-tetrahydrochinolin-6-yl]-3-isobutyryl-6,6-dimethyl-3,6-dihydro-2H-1,3,4-thiadiazin-2-on;
5-[1-(N-Ethyl-m-methoxybenzimidoyl)-1,2,3,4-tetrahydrochinolin-6-yl]-3-isobutyryl-6,6-dimethyl-3,6-dihydro-2H-1,3,4-thiadiazin-2-on;
5-[1-(N-Ethyl-2,4-dimethoxybenzimidoyl)-1,2,3,4-tetrahydrochinolin-6-yl]-3-butyryl-6,6-dimethyl-3,6-dihydro-2H-1,3,4-thiadiazin-2-on;
5-[1-(N-Methyl-p-methoxybenzimidoyl)-1,2,3,4-tetrahydrochinolin-6-yl]-3-butyryl-6,6-dimethyl-3,6-dihydro-2H-1,3,4-thiadiazin-2-on;
5-(1-(N-Ethyl-3,4-dichlorbenzimidoyl)-1,2,3,4-tetrahydrochinolin-6-yl]-3-propionyl-6,6-dimethyl-3,6-dihydro-2H-1,3,4-thiadiazin-2-on;
5-[1-(N-Ethyl-2,4-dichlorbenzimidoyl)-1,2,3,4-tetrahydrochinolin-6-yl]-3-isobutyryl-6,6-dimethyl-3,6-dihydro-2H-1,3,4-thiadiazin-2-on;
5-[1-(N-Isopropyl-m-methoxybenzimidoyl)-1,2,3,4-tetrahydrochinolin-6-yl]-3-isobutyryl-6,6-dimethyl-3,6-dihydro-2H-1,3,4-thiadiazin-2-on;
5-[1-(N-Cyclohexyl-p-methoxybenzimidoyl)-1,2,3,4-tetrahydrochinolin-6yl]-3-isobutyryl-6,6-dimethyl-3,6-dihydro-2H-1,3,4-thiadiazin-2-on;
5-[1-((N-lsopropyl-m-chlorbenzimidoyl)-1,2,3,4-tetrahydrochinolin-6-yl]-3-butyryl-6,6-dimethyl-3,6-dihydro-2H-1,3,4-thiadiazin-2-on;
5-[1-(N-Isopropyl-2-thienylimidoyl)-1,2,3,4-tetrahydrochinolin-6-yl]-3-butyryl-6,6-dimethyl-3,6-dihydro-2H-1,3,4-thiadiazin-2-on;
5-[1-(N-Benzyl-p-methoxybenzimidoyl)-1,2,3,4-tetrahydrochinolin-6-yl]-3-butyryl-6,6-dimethyl-3,6-dihydro-2H-1,3,4-thiadiazin-2-on;
5-[1-(N-Isopropyl-o-methoxybenzimidoyl)-1,2,3,4-tetrahydrochinolin-6yl]-3-butyryl-6,6-dimethyl-3,6-dihydro-2H-1,3,4-thiadiazin-2-on;
5-[1-(N-Isopropyl-m-methoxybenzimidoyl)-1,2,3,4-tetrahydrochinolin-6-yl]-3-butyryl-6,6-dimethyl-3,6-dihydro-2H-1,3,4-thiadiazin-2-on;
5-[1-(N-Isopropyl-2,4-dimethoxybenzimidoyl)-1,2,3,4-tetrahydrochinolin-6-yl]-3-butyryl-6,6-dimethyl-3,6-dihydro-2H-1,3,4-thiadiazin-2-on;
5-[1-(N-Cyclohexyl-p-methoxybenzimidoyl)-1,2,3,4-tetrahydrochinolin-6-yl]-3-butyryl-6,6-dimethyl-3,6-dihydro-2H-1,3,4-thiadiazin-2-on;
5-[1-(N-Isopropyl-3,4-dichlorbenzimidoyl)-1,2,3,4-tetrahydrochinolin-6-yl]-3-propionyl-6,6-dimethyl-3,6-dihydro-2H-1,3,4-thiadiazin-2-on;
5-[1-(N-Isopropyl-2,4-dichlorbenzimidoyl)-1,2,3,4-tetrahydrochinolin-6-yl]-3-isobutyryl-6,6-dimethyl-3,6-dihydro-2H-1,3,4-thiadiazin-2-on.

### Beispiel 8

Analog Beispiel 1 erhält man ausgehend von 5-(1,2,3,4-Tetrahydrochinolin-6-yl)-3,6,6-trimethyl-3,6-dihydro-1,3,4-thiadizin-2-on ("B") [erhältlich aus N-Methyl-hydrazinthioameisensäure-O-ethylester und 6-(-2-Chlor-2-methyl-propionyl)-2-oxo-1,2,3,4-tetrahydrochinolin] durch Umsetzung mit N-[1-N-(p-Methansulfonamidophenyl)-3,4-dimethoxybenzimidoylchlorid das 5-[1-(n-(p-Methansulfonamidophenyl)-3,4-dimethoxyben- zimidoyl)-1,2,3,4-tetrahydrochinolin-6-yl]-3,6,6-trimethyl-3,6-dihydro-2H-1,3,4-thiadiazin-2-on.

Analog erhält man:
ausgehend von "B" durch Umsetzung
mit N-(p-Methansulfonamidophenyl)-4-nitrobenzoesäureimidchlorid:
   5-[1-(N-(p-Methansulfonamidophenyl)-4-nitrobenzimidoyl)-1,2,3,4-tetrahydrochinolin-6-yl]-3,6,6-trimethyl-3,6-dihydro-2H-1,3,4-thiadiazin-2-on;
mit N-(p-Methansulfonamidophenyl)-4-cyanbenzoesäureimidchlorid:
   5-[1-(N-(p-Methansulfonamidophenyl)-4-cyanbenzimidoyl)-1,2,3,4-tetrahydrochinolin-6-yl]-3,6,6-trimethyl-3,6-dihydro-2H-1,3,4-thiadiazin-2-on;
mit N-(p-Methansulfonamidophenyl)-3,4-dimethoxybenzoesäureimidchlorid:
   5-[1-(N-(p-Methansulfonamidophenyl)-3,4-dimethoxyhenzimidoyl)-1,2,3,4-tetrahydrochinolin-6-yl]-3,6,6-trimethyl-3,6-dihydro-2H-1,3,4-thiadiazin-2-on;
mit N-(p-Methansulfonamidophenyl)-4-methansulfonamidobenzoesäureimidchlorid:
   5-[1-(N-(p-Methansulfonamidophenyl)-4-methansulfonamidobenzimidoyl)-1,2,3,4-tetrahydrochinolin-6-yl]-3,6,6-trimethyl-3,6-dihydro-2H-1,3,4-thiadiazin-2-on;
mit N-(p-Methansulfonamidophenyl)-4-nitrobenzoesäureimidchlorid:
   5-[1-(N-(p-Methansulfonamidobenzyl)-4-nitrobenzimidoyl)-1,2,3,4-tetrahydrochinolin-6-yl]-3,6,6-trimethyl-3,6-dihydro-2H-1,3,4-thiadiazin-2-on;
mit N-(p-Methansulfonamidobenzyl)-4-cyanbenzoesäureimidchlorid:
   5-[1-(N-(p-Methansulfonamidobenzyl)-4-cyanbenzimidoyl)-1,2,3,4-tetrahydrochinolin-6-yl]-3,6,6-trimethyl-3,6-dihydro-2H-1,3,4-thiadiazin-2-on;
mit N-(p-Methansulfonamidobenzyl)-3,4-dimethoxybenzoesäureimidchlorid:
   5-[1-(N-(p-Methansulfonamidobenzyl)-3,4-dimethoxybenzimidoyl)-1,2,3,4-tetrahydrochinolin-6-yl]-3,6,6-trimethyl-3,6-dihydro-2H-1,3,4-thiadiazin-2-on;
mit N-(p-Methansulfonamidobenzyl)-4-methansulfonamidobenzoesäureimidchlorid:
   5-[1-(N-(p-Methansulfonamidobenzyl)-4-methansulfonamidobenzimidoyl)-1,2,3,4-tetrahydrochinolin-6-yl]-3,6,6-trimethyl-3,6-dihydro-2H-1,3,4-thiadiazin-2-on;
mit N-[2-(p-Methansulfonamidophenyl)-ethyl]-4-nitrobenzoesäureimidchlorid:
   5-[1-(N-(2-p-Methansulfonamidophenyl)-ethyl)-4-nitrobenzimidoyl)-1,2,3,4-tetrahydrochinolin-6-yl]-3,6,6-trimethyl-3,6-dihydro-2H-1,3,4-thiadiazin-2-on;
mit N-[2-(p-Methansulfonamidophenyl)-ethyl]-4-cyanbenzoesäureimidchlorid:
   5-[1-(N-(2-(p-Methansulfonamidophenyl)-ethyl)-4-cyanbenzimidoyl)-1,2,3,4-tetrahydrochinolin-6-yl]-3,6,6-trimethyl-3,6-dihydro-2H-1,3,4-thiadiazin-2-on;
mit N-[2-(p-Methansulfonamidophenyl)-ethyl]-3,4-dimethoxybenzoesäureimidchlorid:
   5-[1-(N-(2-(p-Methansulfonamidophenyl)-ethyl]-3,4-dimethoxybenzimidoyl)-1,2,3,4-tetrahydrochinolin-6-yl]-3,6,6-trimethyl-3,6-dihydro-2H-1,3,4-thiadiazin-2-on;
mit N-[2-(p-Methansulfonamidophenyl)-ethyl]-4-methansulfonamidobenzoesäureimidchlorid:
   5-[1-(N-(2-(p-Methansulfonamidophenyl)-ethyl)-4-methansulfonamidohenzimidoyl)-1,2,3,4-tetrahydrochinolin-6-yl]-3,6,6-trimethyl-3,6-dihydro-2H-1,3,4-thiadiazin-2-on.

### Beispiel 9

Analog Beispiel 1 erhält man ausgehend von 5-(1,2,3,4-Tetrahydrochinolin-6-yl)-3-methyl-3,6-dihydro-1,3,4-thiadiazin-2-on ("C") [erhältlich aus 1-Methyl-hydrazinthioameisensäure-0-ethyl-ester und 6-(Chlor-acetyl)-1,2,3,4-tetrahydro-chinolin] durch Umsetzung mit N-(p-Methansulfonamidophenyl)-3,4-dimethoxybenzoesäureimidchlorid das 5-[1-N-(p-Methansulfonamido-phenyl)3,4-dimethoxyhenzimidoyl)-1,2,3,4-tetrahydrochinolin-6-yl]-3-methyl-3,6-dihydro-2H-1,3,4-thiadiazin-2-on.

Analog erhält man:
ausgehend von "C" durch Umsetzung
mit N-(p-Methansulfonamidophenyl)-4-nitrobenzoesäureimidchlorid:
   5-[1-(N-(p-Methansulfonamidophenyl)-4-nitrobenzimidoyl)-1,2,3,4-tetrahydrochinolin-6-yl]-3-methyl-3,6-dihydro-2H-1,3,4-thiadiazin-2-on;
mit N-(p-Methansulfonamidophenyl)-4-cycanbenzoesäureimidchlorid:
   5-[1-(N-(p-Methansulfonamidophenyl)-4-cyanbenzimidoyl)-1,2,3,4-tetrahydrochinolin-6-yl]-3-methyl-3,6-dihydro-2H-1,3,4-thiadiazin-2-on;
mit N-(p-Methansulfonamidophenyl)-4-methansulfonamidobenzoesäureimidchlorid:
   5-[1-(N-(p-Methansulfonamidophenyl)-4-methansulfonamidobenzimidoyl)-1,2,3,4-tetrahydrochinolin-6-yl]-3-methyl-3,6-dihydro-2H-1,3,4-thiadiazin-2-on;
mit N-(p-Methansulfonamidophenylbenzyl)-4-nitrobenzoesäureimidchlorid:
   5-[1-(N-(p-Methansulfonamidobenzyl)-4-nitrobenzimidoyl)-1,2,3,4-tetrahydrochinolin-6-yl]-3-methyl-3,6-dihydro-2H-1,3,4-thiadiazin-2-on;
mit N-(p-Methansulfonamidobenzyl)-4-cyanbenzoesäureimidchlorid:
   5-[1-(N-(p-Methansulfonamidobenzyl)-4-cyanbenzimidoyl)-1,2,3,4-tetrahydrochinolin-6-yl]-3-methyl-3,6-dihydro-2H-1,3,4-thiadiazin-2-on;
mit N-(p-Methansulfonamidobenzyl)-3,4-dimethoxybenzoesäureimidchlorid:
   5-[1-(N-(p-Methansulfonamidobenzyl)-3,4-dimethoxybenzimidoyl)-1,2,3,4-tetrahydrochinolin-6-yl]-3-methy-3,6-dihydro-2H-1,3,4-thiadiazin-2-on;
mit N-(p-Methansulfonamidobenzyl)-4-methansulfonamidobenzoesäureimidchlorid:
   5-[1-(N-(p-Methansulfonamidobenzyl)-4-methansulfonamidobenzimidoyl)-1,2,3,4-tetrahydrochinolin-6-yl]-3-methyl-3,6-dihydro-2H-1,3,4-thiadiazin-2-on;
mit N-[2-(p-Methansulfonamidophenyl)-ethyl]-4-nitrobenzoesäureimidchlorid:
   5-[1-(N-(2-(p-Methansulfonamidobenzyl)-ethyl)-4-nitrobenzimidoyl)-1,2,3,4-tetrahydrochinolin-6-yl ]-3-methyl3,6-dihydro-2H-1,3,4-thiadiazin-2-on;
mit N-[2-(p-Methansulfonamidophenyl)-ethyl]-4-cyanbenzoesäureimidchlorid:
   5-[1-(N-2-(p-Methansulfonamidophenyl)-ethyl)-4-cyanbenzimidoyl)-1,2,3,4-tetrahydrochinolin-6-yl]-3-methyl-3,6-dihydro-2H-1,3,4-thiadiazin-2-on;
mit N-[2-(p-Methansulfonamidophenyl-ethyl]-3,4-dimethoxybenzoesäureimidchlorid:
   5-[1-(N-(2-(p-Methansulfonamidophenyl)-ethyl)-3,4-dimethoxybenzimidoyl)-1,2,3,4-tetrahydrochinolin-6-yl]-3-methyl-3,6-dihydro-2H-1,3,4-thiadiazin-2-on;
mit N-[2-(p-Methansulfonamidophenyl)-ethyl]-4-methansulfonamidobenzoesäureimidchlorid:
   5-[1-(N-(2-(p-Methansulfonamidophenyl)-ethyl)-4-methansulfonamidobenzimidoyl)-1,2,3,4-tetrahydrochinolin-6-yl]-3-methyl-3,6-dihydro-2H-1,3,4-thiadiazin-2-on;
mit N-Methyl-4-nitrobenzoesäureimidchlorid:
   5-[1-(N-Methyl-4-nitrobenzimidoyl)-1,2,3,4-tetrahydrochinolin-6-yl]-3-methyl-3,6-dihydro-2H-1,3,4-thiadiazin-2-on;
mit N-Methyl-4-cyanbenzoesäureimidchlorid:
   5-[1-(N-Methyl-4-cyanbenzimidoyl)-1,2,3,4-tetrahydrochinolin-6-yl]-3-methyl-3,6-dihydro-2H-1,3,4-thiadiazin-2-on;
mit N-Methyl-3,4-dimethoxybenzoesäureimidchlorid:
   5-[1-(N-Methyl-3,4-dimethoxybenzimidoyl)-1,2,3,4-tetrahydrochinolin-6-yl]-3-methyl-3,6-dihydro-2H-1,3,4-thiadiazin-2-on;
mit N-Methyl-4-methansulfonamidobenzoesäureimidchlorid:
   5-[1-(N-Methyl-4-methansulfonamidobenzimidoyl)-1,2,3,4-tetrahydrochinolin-6-yl]-3-methyl-3,6-dihydro-2H-1,3,4-thiadiazin-2-on;
mit N-Ethyl-4-nitrobenzoesäuremidchlorid:
   5-[1-(N-Ethyl-4-nitrobenzimidoyl)-1,2,3,4-tetrahydrochinolin-6-yl]-3-methyl-3,6-dihydro-2H-1,3,4-thiadiazin-2-on;
mit N-Ethy-4-cyanbenzoesäureimidchlorid:
   5-[1-(N-Ethyl-4-cyanbenzimidoyl)-1,2,3,4-tetrahydrochinolin-6-yl]-3-methyl-3,6-dihydro-2H-1,3,4-thiadiazin-2-on;
mit N-Ethyl-3,4-dimethoxybenzoesäureimidchlorid:
   5-[1-(N-Ethyl-3,4-dimethoxyhenzimidoyl)-1,2,3,4-tetrahydrochinolin-6-yl]-3-methyl-3,6-dihydro-2H-1,3,4-thiadiazin-2-on;
mit N-Ethyl-4-methansulfonamidobenzoesäureimidchlorid:
   5-[1-(N-Ethyl-4-methansulfonamidobenzimidoyl)-1,2,3,4-tetrahydrochinolin-6-yl]-3-methyl-3,6-dihydro-2H-1,3,4-thiadiazin-2-on.

### Beispiel 10

Analog Beispiel 1 erhält man ausgehend von 5-(2,3-Dihydroindol-5-yl)-3,6,6-trimethyl-3,6-dihydro-1,3,4-thiadiazin-2-on ("D") durch Umsetzung mit N-Ethyl-3,4-dimethoxybenzoesäureimidchlorid das 5-[1-(N-Ethyl-3,4-dimethoxybenzimidoyl)-2,3-dihydroindol-5-yl]-3,6,6-trimethyl-3,6-dihydro-2H-1,3,4-thiadiazin-2-on.

Analog erhält man:
ausgehend von "D" durch Umsetzung
mit N-Ethyl-4-nitrobenzoesäureimidchlorid:
   5-[1-(N-Ethyl-4-nitrobenzimidoyl)-2,3-dihydroindol-5-yl]-3,6,6-trimethyl-3,6-dihydro-2H-1,3,4-thiadiazin-2- on;
mit N-Ethyl-4-cyanbenzoesäureimidchlorid:
   5-[1-(N-Ethyl-4-cyanbenzimidoyl)-2,3-dihydroindol-5-yl]-3,6,6-trimethyl-3,6-dihydro-2H-1,3,4-thiadiazin-2- on;
mit N-Ethyl-3,4-dimethoxybenzoesäureimidchlorid:
   5-[1-(N-Ethyl-3,4-dimethoxyhenzimidoyl)-2,3-dihydroindol-5-yl]-3,6,6-trimethyl-3,6-dihydro-2H-1,3,4-thiadiazin-2-on;
mit N-Ethyl-4-methansulfonamidobenzoesäureimidchlorid:
   5-[1-(N-Ethyl-4-methansulfonamidobenzimidoyl)-2,3-dihydroindol-5-yl]-3,6,6-trimethyl-3,6-dihydro-2H-1,3,4-thiadiazin-2-on;
Analog erhält man ausgehend von 5-(2,3-Dihydroindol-5-yl)-6,6-dimethyl-3,6-dihydro-1,3,4-thiadiazin-2-on durch Umsetzung
mit N-Ethyl-4-nitrobenzoesäureimidchlorid:
   5-[1-(N-Ethyl-4-nitrobenzimidoyl)-2,3-dihydroindol-5-yl]-6,6-dimethyl-3,6-dihydro-2H-1,3,4-thiadiazin-2- on;
mit N-Ethyl-4-cyanbenzoesäureimidchlorid:
   5-[1-(N-Ethyl-4-cyanbenzimidoyl)-2,3-dihydroindol-5-yl]-6,6-dimethyl-3,6-dihydro-2H-1,3,4-thiadiazin-2- on;
mit N-Ethyl-3,4-dimethoxybenzoesäureimidchlorid:
   5-[1-(N-Ethyl-3,4-dimethoxybenzimidoyl)-2,3-dihydroindol-5-yl]-6,6-dimethyl-3,6-dihydro-2H-1,3,4-thiadiazin-2-on;
mit N-Ethyl-4-methansulfonamidobenzoesäureimidchlorid:
   5-[1-(N-Ethyl-4-methansulfonamidobenzimidoyl)-2,3-dihydroindol-5-yl]-6,6-dimethyl-3,6-dihydro-2H-1,3,4-thiadiazin-2-on.

### Beispiel 11

Analog Beispiel 1 erhält man ausgehend von 5-(1,2,3,4-Tetrahydrochinolin-6-yl)-3-carbomethoxy-3,6-dihydro-1,3,4-thiadiazin-2-on ("E") durch Umsetzung mit N-Ethyl-3,4-dimethoxybenzoesäureimidchlorid das 5-[1-(N-Ethyl-3,4-dimethoxy-benzimiddoyl)-1,2,3,4-tetrahydrochinolin-6-yl]-3-carbomethoxy-3,6-dihydro-2H-1,3,4-thiadiazin-2-on.

Analog erhält man:
ausgehend von "E" durch Umsetzung
mit N-Ethyl-4-nitrobenzoesäureimidchlorid:
   5-[1-(N-Ethyl-4-nitrobenzimidoyl)-1,2,3,4-tetrahydrochinolin-6-yl]-3-carbomethoxy-3,6-dihydro-2H-1,3,4-thiadiazin-2-on;
mit N-Ethyl-4-cyanbenzoesäureimidchlorid:
   5-[1-(N-Ethyl-4-cyanbenzimidoyl)-1,2,3,4-tetrahydrochinolin-6-yl]-3-carbomethoxy-3,6-dihydro-2H-1,3,4-thiadiazin-2-on;
mit N-Ethyl-2,4-dimethoxybenzoesäureimidchlorid:
   5-[1-(N-Ethyl-2,4-dimethoxybenzimidoyl)-1,2,3,4-tetrahydrochinolin-6-yl]-3-carbomethoxy-3,6-dihydro-2H-1,3,4-thiadiazin-2-on;
mit N-Ethyl-4-methansulfonamidobenzoesäureimidchlorid:
   5-[1-(N-Ethyl-4-methansulfonamidobenzimidoyl)-1,2,3,4-tetrahydrochinolin-6-yl]-3-carbomethoxy-3,6-dihydro-2H-1,3,4-thiadiazin-2-on.

### Beispiel 12

Analog Beispiel 1 erhält man ausgehend von 5-(1,2,3,4-Tetrahydrochinolin-6-yl)-3-carbomethoxy-6,6-dimethyl-3,6-dihydro-1,3,4-thiadiazin-2-on ("F") durch Umsetzung mit N-Ethyl-3,4-dimethoxybenzoesäurei- midchlorid das 5-[1-(N-Ethyl-3,4-dimethoxy-benzimidoyl)-1,2,3,4-tetrahydrochinolin-6-yl]-3-carbmethoxy-6,6-dimethyl-3,6-dihydro-2H-1,3,4-thiadiazin-2-on.

Analog erhält man:
ausgehend von "F" durch Umsetzung
mit N-Ethyl-4-nitrobenzoesäureimidchlorid:
   5-[1-(N-Ethyl-4-nitrobenzimidoyl)-1,2,3,4-tetrahydrochinolin-6-yl]-3-carbomethoxy-6,6-dimethyl-3,6-dihydro-2H-1,3,4-thiadiazin-2-on;
mit N-Ethyl-4-cyanbenzoesäureimidchlorid:
   5-[1-(N-Ethyl-4-cyanbenzimidoyl)-1,2,3,4-tetrahydrochinolin-6-yl]-3-carbomethoxy-6,6-dimethyl-3,6-dihydro-2H-1,3,4-thiadiazin-2-on;
mit N-Ethyl-2,4-dimethoxybenzoesäureimidchlorid:
   5-[1-(N-Ethyl-2,4-dimethoxybenzimidoyl)-1,2,3,4-tetrahydrochinolin-6-yl]-3-carbomethoxy-6,6-dimethyl-3,6-dihydro-2H-1,3,4-thiadiazin-2-on;
mit N-Ethyl-4-methansulfonamidobenzoesäureimidchlorid:
   5-[1-(N-Ethyl-4-methansulfonamidobenzimidoyl)-1,2,3,4-tetrahydrochinolin-6-yl]-3-carbomethoxy-6,6-dimethyl-3,6-dihydro-2H-1,3,4-thiadiazin-2-on.

Die nachstehenden Beispiele betreffen pharmazeutische Zubereitungen, die Verbindungen der Formel I oder ihre Säureadditionssalze enthalten.

### Beispiel A: Tabletten

Ein Gemisch von 1 kg 5-[1-(N-Ethyl-3,4-dimethoxybenzimidoyl)-1,2,3,4-tetrahydrochinolin-6-yl]-6,6-dimethyl-3,6-dihydro-2H-1,3,4-thiadiazin-2-on, 10kg Lactose, 6 kg mikrokristalline Cellulose, 6 kg Kartoffelstärke, 1 kg Polyvinylpyrrolidon, 0,8 g Talk und 0,1 kg Magnesiumstearat wird in üblicher Weise Zu Tabletten gepreßt, derart, daß jede Tablette 10 mg Wirkstoff enthält.

### Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

### Beispiel C: Kapseln

1 kg 5[1-(N-Isopropyl-3,4-dimethoxybenzimidoyl)-1,2,3,4-tetrahydrochinolin-6-yl)-6,6-dimethyl-3,6-dihydro-2H-1,3,4-thiadiazin-2-on wird in üblicher Weise in Hartgelatinekapseln gefüllt, so daß jede Kapsel 5 mg Wirkstoff enthält.

### Beispiel D: Ampullen

Eine Lösung von 1 kg 5[1-(N-Ethyl-p-fluorbenzimidoyl)-1,2,3,4-tetrahydrochinolin-6-yl]-6,6-dimethyl-3,6-dihydro-2H-1,3,4-thiadiazin-2-on in 30 I 1,2-Propandiol wird steril filtriert, in Ampullen abgefüllt und steril verschlossen. Jede Ampulle enthält 2 mg Wirkstoff.

Analog sind Tabletten, Dragees, Kapseln und Ampullen erhältlich, die einen der übrigen Wirkstoffe der Formel I und/oder ihre physiologisch unbedenklichen Säureadditionssalze enthalten.

Ebenso können die Verbindungen zur Herstellung anderer Formulierungen und Applikationsformen verwendet werden.

## Patentansprüche

1. Thiadiazinonderivate der Formel I 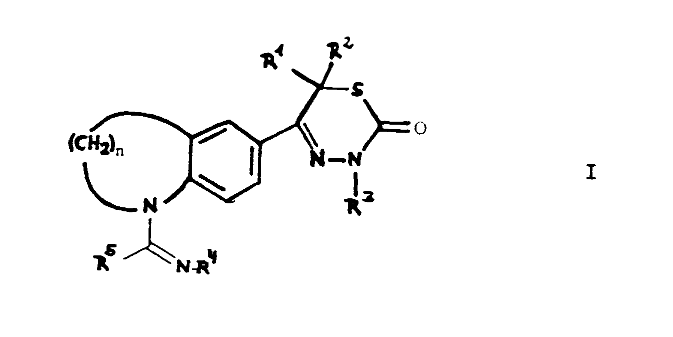
worin
R¹ und R² jeweils unabhängig voneinander H oder A,
_{R}3 H, A oder Ac,
R⁴ H, A, Cycloalkyl mit 3-7 C-Atomen, Ar oder Ar-alk,
_{R}⁵ Ar oder Het,
A Alkyl mit 1-8 C-Atomen,
Ac A-CO-, Ar-CO-, Ar-alk-CO-, A-O-CO- oder A-NH-CO-,
-alk Alkylen mit 1-5 C-Atomen,
Ar einen unsubstituierten oder einen ein-, zwei- oder dreifach durch A, OH, OA, F, Cl, Br, I, SA, SOA, S0₂A, NH₂, NHA, NA₂, NHAc, NHS0₂A, CN oder N0₂ substituierten Phenylrest,
Het einen gesättigten oder ungesättigten 5- oder 6-gliedrigen heterocyclischen Rest mit 1-4 N-, O- und/oder S-Atomen, der ein- oder zweifach durch A, OA, F, Cl, Br, I, OH, N0₂, NH₂, NHA, NA₂, NHAc, NH-S0₂-A, SO-A, S0₂-A, S0₂NH₂ und/oder S0₂NHA, substituiert sein kann
und
n 2, 3 oder 4
bedeuten,
sowie deren Salze.

2.
a)5-[1-(N-Ethyl-4-methoxybenzimidoyl)-1,2,3,4-tetrahydrochinolin-6-yl]-6,6-dimethyl-3,6-dihydro-2H-1,3,4-thiadiazin-2-on;
b) 5-[1-(N-Phenyl-benzimidoyl)-1,2,3,4-tetrahydrochinolin-6-yl]-6,6-dimethyl-3,6-dihydro-2H-1,3,4-thiadiazin-2-on;
c)5-[1-(N-Ethyl-4-chlorbenzimidoyl)-1,2,3,4-tetrahydrochinolin-6-yl]-6,6-dimethyl-3,6-dihydro-2H-1,3,4-thiadiazin-2-on;
d) 5-[1-(N-(2-Phenyl-ethyl)-3,4-dimethoxybenzimidoyl)-1,2,3,4-tetrahydrochinolin-6-yl]-6,6-dimethyl-3,6-dihydro-2H-1,3,4-thiadiazin-2-on;
e)5-[1-(N-Ethyl-2-thienylimidoyl)-1,2,3,4-tetrahydrochinolin-6-yl]-6,6-dimethyl-3,6-dihydro-2H-1,3,4-thiadiazin-2-on.

3. Verfahren zur Herstellung von Thiadiazinonen der Formel I nach Anspruch 1 sowie von deren Salzen, dadurch gekennzeichnet, daß man eine Verbindung der Formel II 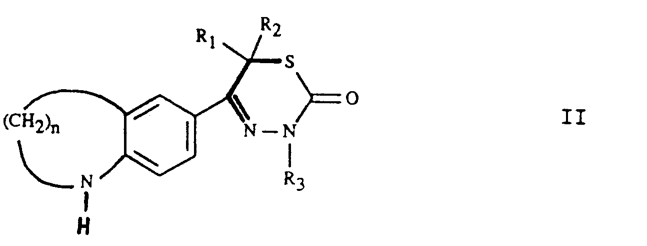
worin
R¹, R², R³ und n die angegebenen Bedeutungen haben, mit einem Imidchlorid der Formel III 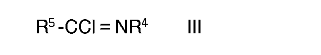
worin
R⁴ und R⁵ die angegebenen Bedeutungen haben,
umsetzt,
oder daß man ein Keton der Formel IV 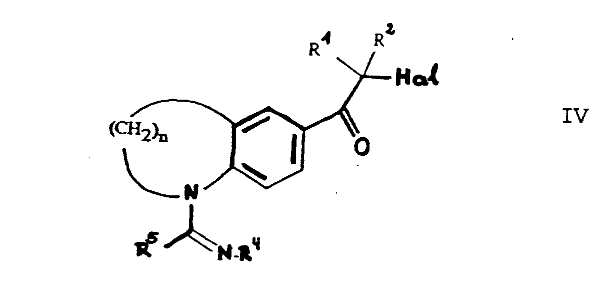
worin
R¹, R², R⁴, R⁵ und n die in Anspruch 1 angegebenen Bedeutungen haben und
Hal Cl, Br oder I bedeutet,
mit einer Verbindung der Formel V 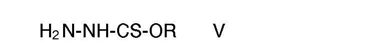
worin
R Alkyl mit 1-5 C-Atomen oder ein Äquivalent eines Metall- oder Ammoniumkations bedeutet, umsetzt,
oder daß man zur Herstellung einer Verbindung der Formel I, worin R⁴ = H ist, eine entsprechende Verbindung, die jedoch anstelle von R⁴ eine Aminoschutzgruppe trägt, mit einem hydrolysierenden oder hydrogenolysierenden Mittel behandelt,
oder daß man zur Herstellung einer Verbindung der Formel I, worin R⁴ = H ist, eine Halogenmagnesiumverbindung der Formel VI 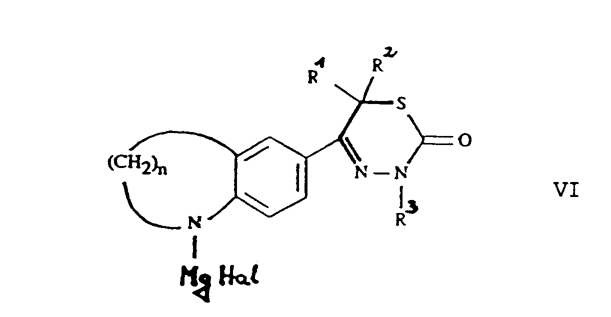
worin
R¹, R², R³, Hal und n die angegebenen Bedeutungen haben,
mit einem Nitril der Formel VII 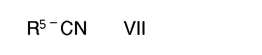
worin
R⁵ die angegebene Bedeutung hat, umsetzt und das erhaltene Produkt anschließend hydrolysiert, oder daß man eine Verbindung der Formel VIII 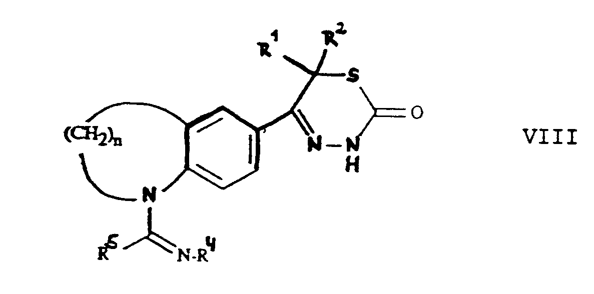
worin
R¹, R², R⁴, R⁵ und n die angegebenen Bedeutungen haben,
mit einem Acyl- oder Alkylderivat der Formel IX 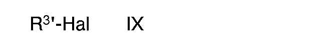
worin
_{R}³' A oder Ac und
Hal die zuvor angegebene Bedeutung hat,
umsetzt,
oder daß man gegebenenfalls in einem Thiadiazinonderivat der Formel I einen oder beide Rest(e) R⁴ und/oder R⁵ in (einen) andere(n) Rest(e) R⁴ und/oder R⁵ umwandelt
und/oder
eine erhaltene Base der Formel I durch Behandlung mit einer Säure in eines ihrer Säureadditionssalze umwandelt.

4. Verfahren zur Herstellung einer pharmazeutischen Zubereitung, dadurch gekennzeichnet, daß man eine Verbindung der Formel I gemäß Anspruch 1 und/oder eines ihrer physiologisch unbedenklichen Salze zusammen mit mindestens einem festen, flüssigen oder halbflüssigen Träger- oder Hilfsstoff in eine geeignete Darreichungsform bringt.

5. Pharmazeutische Zubereitung, gekennzeichnet durch einen Gehalt an mindestens einer Verbindung der Formel I gemäß Anspruch 1 und/oder einem ihrer physiologisch unbedenklichen Salze.

6. Verwendung einer Verbindung der Formel I gemäß Anspruch 1 und/oder eines ihrer physiologisch unbedenklichen Salze bei der Bekämpfung von Krankheiten.

7. Verwendung einer Verbindung der Formel I gemäß Anspruch 1 und/oder eines ihrer physiologisch unbedenklichen Salze zur Herstellung von Arzneimitteln.
